# EUROPEAN PATENT APPLICATION

(11) **EP 4 477 693 A1**
(43) Date of publication of application: **18.12.2024**
(21) Application number: 23785018.5
(22) Date of filing: 06.04.2023
(51) Int. Cl.: C08J 11/02, G01N 31/22, G01N 21/78, G01N 33/44

(54) **METHOD FOR SELECTING ESTER FUNCTIONAL GROUP-CONTAINING POLYMERS FROM MIXTURE OF ESTER FUNCTIONAL GROUP-CONTAINING POLYMERS AND OTHER TYPES OF POLYMERS**

(30) Priority: 06.04.2022 KR 20220042857
(71) Applicant: Korea Research Institute of Chemical Technology, Daejeon 34114 (KR); Re-New System Co., Ltd, Seoul 03929 (KR)
(72) Inventor: CHO, Joungmo, Daejeon 34114 (KR); JO, Ye Rim, Daejeon 34114 (KR); LE, Thi Hong Ngan, Daejeon 34114 (KR); LEE, Jong Yong, Seoul 04036 (KR)
(74) Representative: Isarpatent
(86) International application number: PCT/KR2023/004653
(87) International publication number: WO 2023/195794

(57) **Abstract**

The present invention relates to a method for selecting ester functional group-containing polymers from a polymer mixture of the ester functional group-containing polymers and other types of polymers, and more particularly, to a method wherein foreign substances that only exhibit color in the ester functional group-containing polymers are removed from the polymer mixture by using an extracting agent for extracting the foreign substances and decolorized, and then the decolorized polymers are selectively separated by using the color difference with the other non-decolorized polymers.

## Description

### Technical Field

The present disclosure relates to a method of effectively selecting an ester functional group-containing polymer from a polymer mixture containing the ester functional group-containing polymer and other types of polymers. The present disclosure relates to a technique that allows selecting an ester functional group-containing polymer from a polymer mixture relatively simply and at low cost by using a method of removing only a color-producing foreign substance contained in an ester functional group-containing colored polymer using a chemical extractant.

### Background Art

Synthetic polymers are synthesized substances using raw materials extracted from petroleum. Synthetic polymers are advantageous in that they are inexpensive and durable, as well as being easy to mold and process. Accordingly, synthetic polymers are used to produce various products such as synthetic fibers and plastics. Due to these advantages, synthetic polymer products have been dramatically consumed over several decades in different areas of daily life. However, waste from the synthetic polymer products (,or synthetic polymer waste) is poorly managed and abandoned in the environment. This has been causing environmental pollution, or the waste has been broken down into small pieces and circulating in the ecosystem and accumulated in living things. Ultimately, problems occur when the waste enters the human body again through fine dust, drinking water, and food.

To solve the problem, various research and efforts have been made from the development of new plastic materials with short decomposition cycles in nature to the physical recycling and reprocessing of existing petroleum-based plastics after the chemical decomposition thereof. That efforts are being made to minimize the plastic accumulation or reduce environmental impacts of plastics. Herein, material or physical recycling means reusing the polymer materials themselves or modifying only some of their properties. The material or physical recycling has limited uses because the recycling intervention makes it difficult to manage physical properties or quality. Accordingly, a chemical recycling technique has received a lot of attention. The chemical recycling technique means depolymerizing waste synthetic polymer products discharged after use to produce monomers and use the monomers as raw materials for polymerization to reproduce products of the same quality as the previous materials.

The depolymerization method of synthetic polymers varies depending on the types of synthetic polymers. Thus, waste polymer products containing various components should be classified first as a single substance with uniform polymer structural properties. This is especially true in the case of waste fiber products, as the waste fiber products are often a mixture of various types of fibers or are discharged as synthetic materials.

Ester functional group-containing polymer products may be monomerized through depolymerization. Monomers produced through decomposition theoretically have properties equivalent to the raw materials used in initial polymer synthesis. Depolymerization routes used industrially to recycle PET include hydrolysis, glycolysis, methanolysis, and ammonolysis. Various chemical depolymerization methods are being applied even to complex processes in which these routes are combined by making the use of each process.

Meanwhile, in polymer products, color-producing substances such as dyes or pigments are introduced and used in a more common way to produce color. The color-producing substances have a different structure from polymers. The color-producing substances remain even after depolymerization of polymers and may affect product characteristics, so the substances are considered difficult-to-remove impurities. Thus, it is best to remove these color-producing foreign substances before or after the depolymerization. Dyes as color-producing foreign substances within polymers have a uniformly dissoluble form in most solvents due to their compact particle sizes or molecular form. Thus, dyes are used to dye fibers and leather through a coloring process. The dyes are classified into natural dyes (obtained from plants, animals, or minerals) or into artificial dyes synthesized artificially. The artificial dyes include direct dyes, mordant dyes, reductive dyes, color-producing dyes, disperse dyes, and reactive dyes. The most representative dyes applied to ester functional group-containing polymer materials are azo-based and anthraquinone-based disperse dyes, which may be classified by chemical structure.

An ester functional group-containing colored polymer exists in the complex form of a polymer substrate and a colored dye molecule (i.e., a color-producing foreign substance within the polymer substrate). Thus, it is difficult to separate or remove the color-producing colored molecule through simple contact with water or organic solvents, which makes physical and chemical recycling difficult. Accordingly, the ester functional group-containing colored polymer is known as a waste resource that causes environmental problems since the ester functional group-containing colored polymer is mostly discarded or incinerated.

Therefore, there is a need to develop a very effective and economical separation technique to remove a color-producing foreign substance from the ester functional group-containing colored polymer. For this, it is necessary to first select an ester functional group-containing polymer from a waste polymer mixture discharged.

Conventional separation methods of selecting a polymer include a physical separation method of separating a polymer by density difference, a spectroscopic separation method of separating a polymer on the basis of the absorption, diffusion, and reflection spectrum of light depending on the structure of the polymers, and lastly a chemical separation method through polymer decomposition such as oxidation, reduction, hydrolysis, and electrolysis.

As conventional arts, International Publication Patent No. WO2013-182801 discloses a method of separating a fiber by density difference between polyester and cotton, and Chinese Published Patent CN 104730004 A and Autex Research Journal, 19(2), 201-209 (2019) discloses a method of identifying a fiber by taking advantage of the fact that the diffuse reflection spectrum of light appears differently depending on the structure of the fiber. However, in the conventional arts, the measurement and classification methods that are applied to classify substances are greatly affected by the measurement environment or separation conditions, thereby the accuracy of the methods is low. To increase reliability, several auxiliary facilities and complicated processes are required. Therefore, the conventional arts are poorly useful in terms of efficiency and cost.

### Disclosure

### Technical Problem

The present disclosure is to provide a method of selecting an ester functional group-containing polymer by selectively decolorizing only the ester functional group-containing polymer from a polymer mixture of the ester functional group-containing colored polymer and other types of colored polymers, and then distinguishing the polymer, from which a color-producing foreign substance has been removed, from non-decolorized polymers. In addition, the present disclosure is to provide an extractant for selective decolorizing.

In addition, the present disclosure is to provide a method of selecting all polymers containing colorless and colored ester functional groups in a polymer mixture when the polymers containing colorless and colored ester functional groups are mixed in the polymer mixture.

### Technical Solution

To address the problems, the present disclosure may provide an extractant selectively eluting and removing a color-producing foreign substance that selectively develops a color only in an ester functional group-containing polymer, from a polymer mixture of an ester functional group-containing colored polymer and another type of colored polymer. The extractant may include one or more selected from compounds represented by Formula 1 and compounds represented by Formula 2 below.

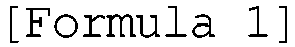

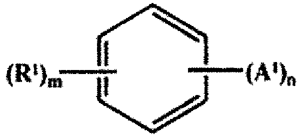

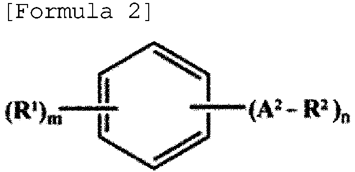

In Formula 1, A¹ may include any one selected from the group consisting of a carboxyl group, an aldehyde group, and a nitrile group. R¹ may include any one selected from the group consisting of a hydroxyl group, carboxyl group, aldehyde group, nitrile group, straight-chain or branched saturated hydrocarbon having 1 to 6 carbon atoms, straight-chain or branched unsaturated hydrocarbon having 1 to 6 carbon atoms, cycloalkyl group having 4 to 6 carbon atoms, and aryl group having 6 to 12 carbon atoms. m may be any integer selected from a range of 0 to 5. When the m is 2 or more, R¹ may be the same or different from each other. n may be any integer selected from a range of 1 to 6. When the n is 2 or more, A¹ may be the same or different from each other.

In addition, in Formula 2, A² may include one or more selected from the group consisting of an ester group, ether group, and carbonyl group. R² may be an alkyl group having 1 to 10 carbon atoms. n may be any integer selected from a range of 1 to 6. When the n is 2 or more, A² and R² may be the same or different from each other. Other definitions of R¹ and m may be the same as in Formula 1 above.

In an embodiment of the present disclosure, the compounds represented by Formula 1 or the compounds represented by Formula 2 may include one or more selected from the group consisting of methoxybenzene, ethoxybenzene, butoxybenzene, 1,2-dimethoxybenzene, 1,3-dimethoxybenzene, 1,4-dimethoxybenzene, 1,2-diethoxybenzene, 1,3-diethoxybenzene, 1,4-diethoxybenzene, 1,2,3-trimethoxybenzene, 1,2,4-trimethoxybenzene, 1,3,5-trimethoxybenzene, 1,2,4-triethoxybenzene, 1,3,5-triethoxybenzene, 1,2,3,4-tetramethoxybenzene, 1,2,3,5-tetramethoxybenzene, 1,2,4,5-tetramethoxybenzene, 1-methoxy-2-methylbenzene, 1-methoxy-3-methylbenzene, 1-methoxy-4-methylbenzene, 1-ethyl-2-methoxybenzene, 1-ethyl-3-methoxybenzene, 1-ethyl-4-methoxybenzene, 1-ethoxy-2-methylbenzene, 1-ethoxy-3-methylbenzene, 1-ethoxy-4-methylbenzene, 1-ethoxy-2-ethylbenzene, 1-ethoxy-3-ethylbenzene, 1-ethoxy-4-ethylbenzene, 1-methoxy-2-prop-2-enylbenzene, 1-methoxy-3-prop-1-enylbenzene, 1-methoxy-3-prop-2-enylbenzene, 1-methoxy-4-prop-2-enylbenzene, 1-methoxy-4-[(E)-prop-1-enyl]benzene (cis), 1-methoxy-4-[(E)-prop-1-enyl]benzene (trans), methyl benzoate, ethyl benzoate, butyl benzoate, propyl benzoate, methyl2-ethoxybenzoate, methyl3-ethoxybenzoate, methyl4-ethoxybenzoate, ethyl2-ethoxybenzoate, ethyl3-ethoxybenzoate, ethyl4-ethoxybenzoate, ethyl(E)3-(4-hydroxy-3,5-dimethoxyphenyl)prop-2-enoate, 2,3-dimethoxy-4-methylbenzoic acid, 2,3-dimethoxybenzoic acid, 2,4-dimethoxybenzoic acid, 2,4-dihydroxybenzoic acid, 2,5-dimethoxy-4-methylbenzoic acid, 2,5-dimethoxybenzoic acid, 2,5-dihydroxybenzoic acid, 2,6-dimethoxy-4-methylbenzoic acid, 2,6-dimethoxybenzoic acid, 2,6-dihydroxybenzoic acid, 2-methoxybenzoic acid, 2-ethoxy-3-ethylbenzoic acid, 2-ethoxybenzoic acid, 2-hydroxy-3-methoxybenzoic acid, 2-hydroxy-4,5-dimethoxybenzoic acid, 2-hydroxy-4,6-dimethoxybenzoic acid, 2-hydroxy-4-methoxybenzoic acid, 2-hydroxy-5-methoxybenzoic acid, 2-hydroxy-6-methoxybenzoic acid, 2-hydroxybenzoic acid, 3-(4-hydroxy-3,5-dimethoxyphenyl)prop-2-enoic acid, 3,4-dimethoxybenzoic acid, 3,4-dihydroxybenzoic acid, 3,5-dimethoxy-4-methylbenzoic acid, 3,5-dimethoxybenzoic acid, 3-methoxybenzoic acid, 3-ethoxy-4-hydroxybenzoic acid, 3-ethoxy-5-ethylbenzoic acid, 3-ethoxybenzoic acid, 3-hydroxy-4,5-dimethoxybenzoic acid, 3-hydroxy-4-methoxybenzoic acid, 3-hydroxy-5-methoxybenzoic acid, 3-hydroxybenzoic acid, 4-methoxybenzoic acid, 4-ethoxy-2-ethylbenzoic acid, 4-ethoxy-3-ethylbenzoic acid, 4-ethoxybenzoic acid, 4-hydroxy-2,6-dimethoxybenzoic acid, 4-hydroxy-2,6-dimethylbenzoic acid, 4-hydroxy-2-methoxybenzoic acid, 4-hydroxy-3,5-dimethoxybenzoic acid, 4-hydroxy-3,5-dimethylbenzoic acid, 4-hydroxy-3-methoxybenzoic acid, 4-hydroxybenzoic acid, ethyl 2-methoxybenzoic acid, ethyl 3-methoxybenzoic acid, ethyl 4-methoxybenzoic acid, 2,3-dimethoxyphenol, 2,4-dimethoxyphenol, 2,5-dimethoxyphenol, 2,6-dimethoxy-4-prop-2-enylphenol, 2,6-dimethoxyphenol, 2-[(E)-prop-1-enyl]phenol, 2-methoxy-4-(prop-1-enyl)phenol, 2-methoxy-4-prop-2-enylphenol, 2- methoxyphenol, 2-ethoxyphenol, 2-tert-butyl-4-methoxyphenol, 3,4-dimethoxyphenol, 3,5-dimethoxyphenol, 3-methoxyphenol, 3-ethoxyphenol, 3-tert-butyl-4-methoxyphenol, 3-prop-1-en-2-ylphenol, 4-(prop-1-en-2-yl)phenol, 4 -(prop-2-en-1-yl)phenol, 4-methoxy-2-[(E)-prop-1-enyl]phenol, 4-methoxyphenol, 4-ethoxyphenol, 4-ethyl-2,3-dimethylphenol, 4-ethyl-2,5-dimethoxyphenol, 4-ethyl-2,6-dimethoxyphenol, 4-tert-butyl-2-methoxyphenol, 1-(2-hydroxy-4,6-dimethylphenyl)ethanone, 1-(4-methoxyphenyl)ethanone 1-(4-hydroxy-2,6-dimethoxyphenyl)ethanone, 1-(4-hydroxy-3,5-dimethoxyphenyl)ethanone, 1-(5-hydroxy-2,4-dimethylphenyl)ethanone, 1-phenylbutan-1-one 1-phenylethanone, 1-phenylpropan-1-one 2,3-dihydroxybenzaldehyde, 2,4-dihydroxybenzaldehyde, 2,5-dihydroxybenzaldehyde, 2-methoxybenzaldehyde, 2-methylbenzaldehyde, 2-butoxybenzaldehyde, 2-ethoxybenzaldehyde, 2-hydroxy-3,5-dimethoxybenzaldehyde, 2-hydroxy-3-methoxybenzaldehyde, 2-hydroxy-4,6-dimethoxybenzaldehyde, 2-hydroxy-4-methoxybenzaldehyde, 2-hydroxy-5-methoxybenzaldehyde, 2-hydroxy-6-methoxybenzaldehyde, 2-hydroxybenzaldehyde, 3,4-dihydroxybenzaldehyde, 3,5-dihydroxybenzaldehyde, 3-methoxybenzaldehyde, 3-methylbenzaldehyde, 3-butoxybenzaldehyde, 3-ethoxy-2-hydroxybenzaldehyde, 3-ethoxybenzaldehyde, 3-hydroxy-2-methoxybenzaldehyde, 3-hydroxy-4-methoxybenzaldehyde, 3-hydroxy-5-methoxybenzaldehyde, 3-hydroxybenzaldehyde, 4-methoxybenzaldehyde, 4-methylbenzaldehyde, 4-butoxybenzaldehyde, 4-ethoxybenzaldehyde, 4-hydroxy-2,6-dimethoxybenzaldehyde, 4-hydroxy-2-methoxybenzaldehyde, 4-hydroxy-3,5-dimethoxybenzaldehyde, 4-hydroxy-3-methoxybenzaldehyde, 4-hydroxybenzaldehyde, benzaldehyde, 2,3-dihydroxybenzonitrile, 2,4-dihydroxybenzonitrile, 2,5-dihydroxybenzonitrile, 2,6-dihydroxybenzonitrile, 2-methoxybenzonitrile, 2-methylbenzonitrile, 2-hydroxybenzonitrile, 3,4-dihydroxybenzonitrile, 3-methoxybenzonitrile, 3-methylbenzonitrile, 3-hydroxybenzonitrile, 4-methoxybenzonitrile, 4-methylbenzonitrile, 4-acetylbenzonitrile, 4-hydroxybenzonitrile, and benzonitrile.

In addition, the present disclosure may provide a method of selecting an ester functional group-containing polymer from a colored polymer mixture of the ester functional group-containing colored polymer and other types of colored polymers. The method may include: (a) selectively decolorizing an ester functional group-containing colored polymer by bringing a polymer mixture of the ester functional group-containing colored polymer and other types of colored polymers into contact with an extractant that extracts a color-producing foreign substance; and (b) separating the decolorized polymer from the non-decolorized polymers in the step (a) by color difference.

In another embodiment of the present disclosure, the ester functional group-containing polymer may have a fiber or fabric form. The fabric may be an ester functional group-containing polyester fiber alone; or a blended fiber including an ester functional group-containing polyester fiber and one or more fibers selected from the group consisting of polyethylene, polypropylene, polystyrene, and polyvinyl chloride, cotton, linen, wool, rayon, acetate, acrylic, nylon, and spandex.

In a further embodiment of the present disclosure, the colored polymers are polymers colored by a foreign substance that produces one or more colors. When the color space coordinate values (L*a*b*) for the polymer mixture measured using a spectrophotometer are converted to two-dimensional (or plane) coordinate values (L*X*), the polymers that satisfy coordinate value criteria "L* of 60 or more and X* of 50 or less" may be excluded. Herein, X* represents the square root of the sum of the squares of each of the a* and b* values.

In a yet further embodiment of the present disclosure, the decolorizing process in the step (a) may be carried out in a temperature range of 70°C to 200°C.

In a still yet further embodiment of the present disclosure, the decolorized polymer may satisfy coordinate value criteria "L* of 60 or more and X* of 50 or less" when the color space coordinate values (L*a*b*) for the decolorized polymer in the step (b) are converted to two-dimensional (or plane) coordinate values (L*X*).

The present disclosure may further provide a method of selecting a colorless ester functional group-containing polymer from a polymer mixture when the polymer mixture contains a colorless ester functional group-containing polymer. The method may include: (i) selectively dyeing only ester functional group-containing polymers by bringing a polymer mixture of the ester functional group-containing polymers and other types of polymers into contact with a color-producing foreign substance; (ii) excluding the colorless polymers from the polymer mixture by color difference after the step (i); (iii) selectively decolorizing only an ester functional group-containing colored polymers by bringing the mixture obtained after the step (ii) into contact with an extractant that extracts a color-producing foreign substance; and (iv) separating the decolorized polymers and non-decolorized polymers from each other by color difference.

In a still yet further embodiment of the present disclosure, the extractant in the step (iii) may include one or more selected from the compounds represented by Formula 1 and the compounds represented by Formula 2.

In a still yet further embodiment of the present disclosure, in the step (ii), the polymers that satisfy the coordinate value criteria "L* of 60 or more and X* of 50 or less" may be excluded when color space coordinate values (L*a*b*) of the polymers are converted to two-dimensional coordinate values (L*X*). In the step (iii), the decolorized polymers that satisfy the coordinate value criteria "L* of 60 or more and X* of 50 or less" may be selected when the color space coordinate values (L*a*b*) of the decolorized polymers are converted to two-dimensional coordinate values (L*X*) .

In a still yet further embodiment of the present disclosure, the waste extractant, which is a mixture of the extractant and the color-producing foreign substance eluted during the decolorizing in the step (iii) may be recovered and used during the dyeing in the step (i).

### Advantageous Effects

The present disclosure is effective in that a color-producing foreign substance that only works in an ester functional group-containing polymer is removed in the ester functional group-containing polymer from a polymer mixture of the ester functional group-containing polymer and other types of polymers, thereby the ester functional group-containing polymer can be easily selected by color difference from the polymer mixture which includes the decolorized ester functional group-containing polymer and other types of non-decolorized colored polymers.

In addition, the extractant according to the present disclosure is a slightly hazardous solvent to the human body. Since the extractant does not cause serious physical/chemical deformation to polymers other than ester functional group-containing polymers and acts only on the ester functional group-containing polymer and quickly elutes colored substances at relatively low temperatures, the use of the extractant in the decolorizing process is simple, economical, and environmentally friendly.

In addition, a waste extractant is recovered after having been used in the decolorizing process and reused in the initial dyeing process of the entire process according to the present disclosure. Thus, it provides the effect of minimizing waste liquid generation in the process itself and improving process economics.

### Description of Drawings

FIG. 1 shows the effect of an extractant according to embodiments of the present disclosure, showing decolorized fibers obtained by bringing respective colored fibers of embodiments into direct contact with anisole as an extractant;
FIG. 2 shows a comparison before and after decolorizing treatment with the two-dimensional coordinate values (L*X*) for fibers converted from the color space coordinate values (L*a*b*) according to embodiments of the present disclosure;
FIG. 3 shows the spectral spectra of decolorized polyester fibers according to embodiments of the present disclosure for comparison depending on the fiber color;
FIG. 4 shows comparisons between the navy polyester fiber of Raw Material 2 and decolorized polyester fibers in terms of a decolorization effect and fiber color change after solvent treatment when a decolorizing process of the navy polyester fiber of Raw Material 2 and decolorized polyester fibers being brought into direct contact with respective solvents was carried out with a solvent containing one or more selected from the compounds represented by Formula 1 and the compounds represented by Formula 2 according to the present disclosure, or with other solvents;
FIG. 5 shows the experiment results according to comparative embodiments of the present disclosure, showing the values representing the decolorization effects of respective solvents at two-dimensional coordinates (L*X*) after the respective solvents had been brought into contact with the navy polyester fibers of Raw Material 2;
FIG. 6 shows ¹³C NMR spectra for mixtures of an ester functional group-containing polymer and an extractant of the present disclosure at different concentrations;
FIG. 7 shows ¹³C NMR spectra for mixtures of an ester functional group-containing polymer and each of the extractants other than that of the present disclosure at different concentrations;
FIG. 8 shows ¹H NMR spectra for mixtures of an ester functional group-containing polymer and an extractant of the present disclosure at different concentrations;
FIG. 9 shows ¹H NMR spectra for mixtures of an ester functional group-containing polymer and each of the extractants other than that of the present disclosure at different concentrations;
FIG. 10 shows the experiment results, showing decolorization effects of the extractant of an embodiment and morpholine of another comparative embodiment, respectively, to compare after they were brought into contact with the navy polyester fiber of Raw Material 2;
FIG. 11 shows decolorization effects when 1-methylpyrrolidin-2-one and methylsulfinylmethane were used as extractants on ester functional group-containing polymers and other colored fibers;
FIG. 12 is photographs of colored fiber mixtures before and after extractant treatment using a Soxhlet extractor, and a photograph of only the decolorized fibers separated;
FIG. 13 shows the spectral spectra of the fibers that satisfy coordinate value criteria among decolorized fibers using a Soxhlet extractor according to embodiments of the present disclosure;
FIG. 14 shows the spectral spectra of colored fibers other than an ester functional group-containing polymer, and the spectral spectra of colorless fibers with comparisons of spectral spectra depending on fiber color;
FIG. 15 is photographs of the fibers dyed according to a further embodiment and comparative embodiments of the present disclosure;
FIG. 16 shows the two-dimensional coordinate values (L*X*) for the fibers converted from the color space coordinate values (L*a*b*), the fibers having been dyed according to the further embodiment and comparative embodiments of the present disclosure;
FIG. 17 shows the two-dimensional coordinate values (L*X*) for the fibers converted from the color space coordinate values (L*a*b*), the fibers having been dyed according to a yet further embodiment and comparative embodiments of the present disclosure; and
FIG. 18 shows a fiber color change when the fiber is re-dyed with a waste extractant solution obtained during the decolorizing process, and then the fiber is decolorized again according to a still yet further embodiment of the present disclosure.

### Best Mode

Unless otherwise defined, all technical and scientific terms used in this specification have the same meaning as commonly understood by those skilled in the art to which the present disclosure pertains. In general, the nomenclature used herein is well-known and commonly used in the art.

Throughout this specification, when a part "includes" a certain component, this means that it may further include other components rather than excluding other components unless specifically stated to the contrary.

In addition, throughout this specification, a colorless polymer refers to a polymer that does not contain a color-producing foreign substance and therefore has no color. The colorless polymer is a white or transparent polymer. A colored polymer refers to a polymer that has a color other than white because the colored polymer contains a color-producing foreign substance.

Hereinafter, a method of selecting and separating only ester functional group-containing colored polymers from a colored polymer mixture of the ester functional group-containing colored polymer and other types of colored polymers will be described in detail according to the present disclosure.

The method includes: (a) decolorizing only an ester functional group-containing colored polymer by bringing a colored polymer mixture of the ester functional group-containing colored polymer and other types of colored polymers into contact with an extractant including one or more selected from compounds represented by Formula 1 and compounds represented by Formula 2 below; and (b) separating the decolorized polymer from the non-decolorized polymers in the step (a) by color difference.

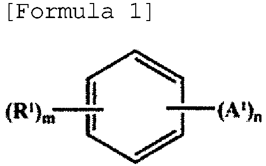

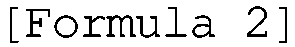

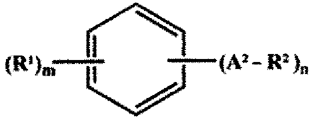

In Formula 1, A¹ may include any one selected from the group consisting of an ether group, an aldehyde group, and a carboxyl group. R¹ may include any one selected from the group consisting of a hydroxyl group, carboxyl group, aldehyde group, nitrile group, straight-chain or branched saturated hydrocarbon having 1 to 6 carbon atoms, straight-chain or branched unsaturated hydrocarbon having 1 to 6 carbon atoms, cycloalkyl group having 4 to 6 carbon atoms, and aryl group having 6 to 12 carbon atoms. m may be any integer selected from a range of 0 to 5. When the m is 2 or more, R¹ may be the same or different from each other. n may be any integer selected from a range of 1 to 6. When the n is 2 or more, A¹ may be the same or different from each other.

In addition, in Formula 2, A² may include one or more selected from an ester group, ether group, and carbonyl group. R² may be an alkyl group having 1 to 10 carbon atoms. n may be any integer selected from a range of 1 to 6. When the n is 2 or more, A² and R² may be the same or different from each other. Other definitions of R¹ and m may be the same as in Formula 1 above.

The extractant in the step (a) includes one or more selected from the compounds represented by Formula 1 and the compounds represented by Formula 2. The compounds have one or more functional groups connected to the aromatic ring through a linker. The functional groups experience a pi-pi interaction (π-π interaction) or a hydrogen bonding force with the ester functional group-containing polymers.

According to the present disclosure, the extractant selectively removes a color-producing substance only from an ester functional group-containing colored polymer in a mixture of various types of polymers. The extractant has no significant effect on changes in the color and shape of other types of polymers. Thus, the extractant may selectively works on removing the color-producing foreign substance from ester functional group-containing colored polymer in a mixture of various types of polymers and be involved in decolorizing.

The compounds represented by Formula 1 or the compounds represented by Formula 2 according to the present disclosure may include, but are not limited to, one or more selected from the group consisting of methoxybenzene, ethoxybenzene, butoxybenzene, 1,2-dimethoxybenzene, 1,3-dimethoxybenzene, 1,4-dimethoxybenzene, 1,2-diethoxybenzene, 1,3-diethoxybenzene, 1,4-diethoxybenzene, 1,2,3-trimethoxybenzene, 1,2,4-tri methoxybenzene, 1,3,5-trimethoxybenzene, 1,2,4-triethoxybenzene, 1,3,5-triethoxybenzene, 1,2,3,4-tetramethoxybenzene, 1,2,3,5-tetramethoxybenzene, 1,2,4,5-tetramethoxybenzene, 1-methoxy-2-methylbenzene, 1-methoxy-3-methylbenzene, 1-methoxy-4-methylbenzene, 1-ethyl-2-methoxybenzene, 1-ethyl-3-methoxybenzene, 1-ethyl-4-methoxybenzene, 1-ethoxy-2-methylbenzene, 1-ethoxy-3-methylbenzene, 1-ethoxy-4-methylbenzene, 1-ethoxy-2-ethylbenzene, 1-ethoxy-3-ethylbenzene, 1-ethoxy-4-ethylbenzene, 1-methoxy-2-prop-2-enylbenzene, 1-methoxy-3-prop-1-enylbenzene, 1-methoxy-3-prop-2-enylbenzene, 1-methoxy-4-prop-2-enylbenzene, 1-methoxy-4-[(E)-prop-1-enyl]benzene (cis), 1-methoxy-4-[(E)-prop-1-enyl]benzene (trans), methyl benzoate, ethyl benzoate, butyl benzoate, propyl benzoate, methyl 2-ethoxybenzoate, methyl 3-ethoxybenzoate, methyl 4-ethoxybenzoate, ethyl 2-ethoxybenzoate, ethyl 3-ethoxybenzoate, ethyl 4-ethoxybenzoate, ethyl(E)3-(4-hydroxy-3,5-dimethoxyphenyl) prop-2-enoate, 2,3-dimethoxy-4-methylbenzoic acid, 2,3-dimethoxybenzoic acid, 2,4-dimethoxybenzoic acid, 2,4-dihydroxybenzoic acid, 2,5-dimethoxy-4-methylbenzoic acid, 2,5-dimethoxybenzoic acid, 2,5-dihydroxybenzoic acid, 2,6-dimethoxy-4-methylbenzoic acid, 2,6-dimethoxybenzoic acid, 2,6-dihydroxybenzoic acid, 2-methoxybenzoic acid, 2-ethoxy-3-ethylbenzoic acid, 2-ethoxybenzoic acid, 2-hydroxy-3-methoxybenzoic acid, 2-hydroxy-4,5-dimethoxybenzoic acid, 2-hydroxy-4,6-dimethoxybenzoic acid, 2-hydroxy-4-methoxybenzoic acid, 2-hydroxy-5-methoxybenzoic acid, 2-hydroxy-6-methoxybenzoic acid, 2-hydroxybenzoic acid, 3-(4-hydroxy-3,5-dimethoxyphenyl)prop-2-enoic acid, 3,4-dimethoxybenzoic acid, 3,4-dihydroxybenzoic acid, 3,5-dimethoxy-4-methylbenzoic acid, 3,5-dimethoxybenzoic acid, 3-methoxybenzoic acid, 3-ethoxy-4-hydroxybenzoic acid, 3-ethoxy-5-ethylbenzoic acid, 3-ethoxybenzoic acid, 3-hydroxy-4,5-dimethoxybenzoic acid, 3-hydroxy-4-methoxybenzoic acid, 3-hydroxy-5-methoxybenzoic acid, 3-hydroxybenzoic acid, 4-methoxybenzoic acid, 4-ethoxy-2-ethylbenzoic acid, 4-ethoxy-3-ethylbenzoic acid, 4-ethoxybenzoic acid, 4-hydroxy-2,6-dimethoxybenzoic acid, 4-hydroxy-2,6-dimethylbenzoic acid, 4-hydroxy-2-methoxybenzoic acid, 4-hydroxy-3,5-dimethoxybenzoic acid, 4-hydroxy-3,5-dimethylbenzoic acid, 4-hydroxy-3-methoxybenzoic acid, 4-hydroxybenzoic acid, ethyl 2-methoxybenzoic acid, ethyl 3-methoxybenzoic acid, ethyl 4-methoxybenzoic acid, 2,3-dimethoxyphenol, 2,4- dimethoxyphenol, 2,5-dimethoxyphenol, 2,6-dimethoxy-4-prop-2-enylphenol, 2,6-dimethoxyphenol, 2-[(E)-prop-1-enyl]phenol, 2-methoxy-4-(prop-1-enyl)phenol, 2-methoxy-4-prop-2-enylphenol, 2-methoxyphenol, 2-ethoxyphenol, 2-tert-butyl-4-methoxyphenol, 3,4-dimethoxyphenol, 3,5-dimethoxyphenol, 3-methoxyphenol, 3-ethoxyphenol, 3-tert-butyl-4-methoxyphenol, 3-prop-1-en-2-ylphenol, 4-(prop-1-en-2-yl)phenol, 4-(prop-2-en-1-yl)phenol, 4-methoxy-2-[(E)-prop-1-enyl]phenol, 4-methoxyphenol, 4-ethoxyphenol, 4-ethyl-2,3-dimethylphenol, 4-ethyl-2,5-dimethoxyphenol, 4-ethyl-2,6-dimethoxyphenol, 4-tert-butyl-2-methoxyphenol, 1-(2-hydroxy-4,6-dimethylphenyl)ethanone, 1-(4-methoxyphenyl)ethanone 1-(4-hydroxy-2,6-dimethoxyphenyl)ethanone, 1-(4-hydroxy-3,5-dimethoxyphenyl)ethanone, 1-(5-hydroxy-2,4-dimethylphenyl)ethanone, 1-phenylbutan-1-one 1-phenylethanone, 1-phenylpropan-1-one 2,3-dihydroxybenzaldehyde, 2,4-dihydroxybenzaldehyde, 2,5-dihydroxybenzaldehyde, 2-methoxybenzaldehyde, 2-methylbenzaldehyde, 2-butoxybenzaldehyde, 2-ethoxybenzaldehyde, 2-hydroxy-3,5-dimethoxybenzaldehyde, 2-hydroxy-3-methoxybenzaldehyde, 2-hydroxy-4,6-dimethoxybenzaldehyde, 2-hydroxy-4-methoxybenzaldehyde, 2-hydroxy-5-methoxybenzaldehyde, 2-hydroxy-6-methoxybenzaldehyde, 2-hydroxybenzaldehyde, 3,4-dihydroxybenzaldehyde, 3,5-dihydroxybenzaldehyde, 3-methoxybenzaldehyde, 3-methylbenzaldehyde, 3-butoxybenzaldehyde, 3-ethoxy-2-hydroxybenzaldehyde, 3-ethoxybenzaldehyde, 3-hydroxy-2-methoxybenzaldehyde, 3-hydroxy-4-methoxybenzaldehyde, 3-hydroxy-5-methoxybenzaldehyde, 3-hydroxybenzaldehyde, 4-methoxybenzaldehyde, 4-methylbenzaldehyde, 4-butoxybenzaldehyde, 4-ethoxybenzaldehyde, 4-hydroxy-2,6-dimethoxybenzaldehyde, 4-hydroxy-2-methoxybenzaldehyde, 4-hydroxy-3,5-dimethoxybenzaldehyde, 4-hydroxy-3-methoxybenzaldehyde, 4-hydroxybenzaldehyde, benzaldehyde, 2,3-dihydroxybenzonitrile, 2,4-dihydroxybenzonitrile, 2,5-dihydroxybenzonitrile, 2,6-dihydroxybenzonitrile, 2-methoxybenzonitrile, 2-methylbenzonitrile, 2-hydroxybenzonitrile, 3,4-dihydroxybenzonitrile, 3-methoxybenzonitrile, 3-methylbenzonitrile, 3-hydroxybenzonitrile, 4-methoxybenzonitrile, 4-methylbenzonitrile, 4-acetylbenzonitrile, 4-hydroxybenzonitrile, and benzonitrile.

In addition, among the compounds represented by Formula 1 and the compounds represented by Formula 2, a compound present in a liquid form due to its lower melting point than an extraction temperature may be used for an extractant, which removes the color-producing foreign substance from the ester functional group-containing colored polymer according to the present disclosure. A compound with low viscosity and fluidity is useful since it allows the color-producing foreign substance to diffuse fast.

The ester functional group-containing colored polymer may be a polymer formed by condensation polymerization of dicarboxylic acid and di-alcohol. Herein, the dicarboxylic acid may include any one selected from the group consisting of terephthalic acid, naphthalene dicarboxylic acid, diphenyl dicarboxylic acid, diphenyl ether dicarboxylic acid, diphenyl sulfone dicarboxylic acid, diphenoxyethane dicarboxylic acid, succinic acid, adipic acid, sebacic acid, azelaic acid, decanedicarboxylic acid, cyclohexanedicarboxylic acid, trimellitic acid, and pyromellitic acid, and combinations thereof. The di-alcohol may include any one selected from the group consisting of ethylene glycol, trimethylene glycol, 1,2-propanediol, tetramethylene glycol, neopentyl glycol, hexamethylene glycol, decanemethylene glycol, dodecamethylene glycol, 1,4-cyclohexanedimethanol, diethylene glycol, triethylene glycol, tetraethylene glycol, polyethylene glycol, dipropylene glycol, tripropylene glycol, tetrapropylene glycol, polypropylene glycol, di(tetramethylene) glycol, tri(tetramethylene) glycol, polytetramethylene glycol, pentaerythritol, and 2,2-bis(4-β-hydroxyethoxyphenyl)propane, and combinations thereof.

For example, the ester functional group-containing colored polymer may include any one selected from the group consisting of polyethylene terephthalate (PET), polypropylene terephthalate (PPT), polyglycolide or polyglycolic acid (PGA), polylactic acid (PLA), polycaprolactone (PCL), polyhydroxyalkanoate (PHA), polyhydroxybutyrate (PHB), polyethylene adipate (PEA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), and vectran, and combinations thereof.

The most common ester functional group-containing colored polymer is polyethylene terephthalate. At this point, starting substances to prepare the polymer are terephthalic acid or a derivative monomer thereof, and ethylene glycol.

The ester functional group-containing polymer may have an ester functional group alone in its form or may have a mixture of an ester functional group-containing polymer and other polymers in a mixed resin form. For example, in the form of the ester functional group-containing polymer, present may be a polymer mixture further including one or more selected from plastic materials (for example, polyethylene, high-density polyethylene, low-density polyethylene, polypropylene, polystyrene, and polyvinyl chloride) and polymers (for example, cotton, hemp, wool, rayon, acetate, acrylic, nylon, and spandex). For reference, other polymers mixed with the polyester functional group-containing polymer are merely listed examples and are not limited thereto.

In addition, the ester functional group-containing polymer may be all or part of the materials molded into a film or bottle. The ester functional group-containing polymer may be a staple fiber, a long filament, or a mixture thereof for clothing or industrial purposes and may be in the form of yarn, amorphous material, or fabric.

In the step (a), the extractant is brought into direct contact with the colored polymer mixture to elute and remove a colored foreign substance only in the ester functional group-containing polymer from the colored polymer mixture. The decolorizing process in the step (a) may be repeated two or more times.

The step (a) may be performed in a temperature range of 70°C to 200°C, but may be more preferably performed in a temperature range of 120°C to 150°C. When the temperature is less than 70°C, the elution rate may proceed slowly and the extraction effect may be limited, and when the temperature exceeds 200°C, energy consumption may be very high.

In addition, in the step (a), an extraction mixed solution containing the color-producing foreign substance is heated, and then a vaporized extractant obtained therefrom is condensed and cooled to re-supply by implementing continuous reflux. Afterward, the extractant in a liquid form is maintained in continuous contact with the ester functional group-containing polymer. This method may be applied to effectively remove a color-producing foreign substance.

To reflux only the extractant in the extraction mixed solution containing the color-producing foreign substance, the temperature of the heating unit may be maintained close to the boiling point of the extractant. A contact temperature (or extraction temperature) between the polymers and the extractant may be determined by the amount of heat applied to the heating unit and the reflux rate of the extractant. This may also affect the performance of extraction. It may be preferable to maintain the contact temperature between the polymers and the extractant to be approximately 0°C to 50°C lower than the boiling point of the extractant when extraction is carried out.

After the color-producing foreign substance is eluted from the ester functional group-containing colored polymer with the use of the extractant, the extractant may be recovered from the extraction mixed solution of the extractant and the color-producing foreign substance by one or more methods selected from evaporation and distillation.

In the step (b), the decolorized polymer and non-decolorized polymers are distinguished by color difference. To detect the color difference, in addition to spectrophotometry, a detector may be used to simply identify whether the dye has been removed on the basis of the brightness or saturation of the color. Color coordinate values such as RGB, HSV, CMYK, or Lab may also be used.

More preferably, the color coordinate value to identify whether or not the material is decolorized may be based on a specular component included (SCI) measurement method, which covers both specular light and diffuse light.

At this point, depending on the measured value and the criteria for determining decolorization, a process may be configured to detect and automatically select decolorized polymers. As described above, when the extractant including one or more selected from the compounds represented by Formula 1 and the compounds represented by Formula 2 according to the present disclosure is applied to the polymer mixture, decolorization occurs only in an ester functional group-containing polymer, thereby only the ester functional group-containing polymer may be selectively distinguished.

The selected polymers may also be used in the recycling industry since difficult-to-purify color contaminants (for example, dyes) are removed in the process of selecting only polymer resins containing highly concentrated ester functional groups in a waste polymer resin mixture. For example, when the selected decolorized polymers are applied to the depolymerization reaction process of the ester functional group-containing polymer, it is possible to produce high-quality monomers without having to undergo a high-cost purification process.

In a still yet further embodiment of the present disclosure, a process of dyeing the ester functional group-containing polymer is further included into a selecting method when the polymer mixture includes an ester functional group-containing colorless polymer, thereby the method of selectively decolorizing an ester functional group-containing polymer may also be applied to a colorless polymer.

The method includes: (i) selectively dyeing only ester functional group-containing polymers by bringing a polymer mixture of the ester functional group-containing polymers and other types of polymers into contact with a color-producing foreign substance; (ii) excluding the colorless polymers from the polymer mixture by color difference after the step (i); (iii) selectively decolorizing only the ester functional group-containing colored polymers by bringing the mixture obtained after the step (ii) into contact with an extractant that extracts a color-producing foreign substance; and (iv) separating decolorized polymers and non-decolorized polymers from each other by color difference.

The step (i) involves turning the ester functional group-containing colorless polymers into colored ones by dyeing the polymers. For this, a polymer resin mixture including colorless polymer resins containing colorless ester functional groups is brought into contact with a disperse dye. At this point, the dye is required to selectively color only the ester functional group-containing polymers. The dye may include, for example, an azo-based or anthraquinone-based disperse dye. The dye for coloring may include a new, unused dye, but the dye may include a waste dye already extracted from ester functional group-containing colored polymer fibers by using the extractant of the present disclosure.

The step (ii) involves excluding the colorless polymers included in the polymer resin mixture. The ester functional group-containing colorless polymers included in the polymer mixture are dyed in the step (i), so there are no ester functional group-containing colorless polymers in the step (ii). However, some ester functional group-containing colorless polymers may exist. Accordingly, by excluding the colorless polymers in the step (ii) by color difference, only colored polymers are introduced into the step (iii).

Next, one or more of the extractants including one or more selected from the compounds represented by Formula 1 and the compounds represented by Formula 2 are used in the step (iii), thereby only ester functional group-containing colored polymers are selectively decolorized among the polymer mixture. The step (iv) involves selecting, by color difference, the polymers from which dyes have been removed, thereby only ester functional group-containing polymers may be selectively separated from a mixture of different types of polymers.

Hereinafter, details of the present disclosure process will be described through examples, comparative examples, and experimental examples. This is a representative example related to the present disclosure, and this alone does not limit the application scope of the present disclosure.

### Ester Functional Group-Containing Polymer Raw Materials

### Raw Material 1

An undyed plain polyester fiber fabric was cut and prepared as a colorless polymer raw material having a square size of approximately 3 cm on one side.

### Raw Material 2

A plain polyester fiber fabric (dye weight ratio: 1.5% o.w.f.) that had been dyed navy using disperse dye (dye: Dystar Dianix Blue E-R 150%; C.I. Disperse Blue 56) was cut and prepared as a colored polymer raw material having a square size of approximately 3 cm on one side.

### Raw Material 3

A plain polyester fiber fabric (dye weight ratio: 1.5% o.w.f.) that had been dyed red using an azo-based disperse dye (C.I. Disperse Red 1) was cut and prepared as a colored polymer raw material having a square size of approximately 3 cm on one side.

### Raw Material 4

A plain polyester fiber fabric (dye weight ratio: 1.5% o.w.f.) that had been dyed red using an anthraquinone-based disperse dye (C.I. Disperse Red 9) was cut and prepared as a colored polymer raw material having a square size of approximately 3 cm on one side.

### Raw Material 5

A blended fabric (dye weight ratio: 1.5% o.w.f.) that had been dyed black using a disperse dye (dye: Dystar Dianix Deep Black Plus), the blended fabric containing 82% polyester fiber and 18% spandex was cut and prepared as a colored polymer raw material having a square size of approximately 3 cm on one side.

### Raw Materials Other Than Ester Functional Group-Containing Polymers

### Raw Material 6

An undyed plain nylon fiber fabric was cut and prepared as a colorless polymer raw material having a square size of approximately 3 cm on one side.

### Raw Material 7

A plain nylon fiber fabric (dye weight ratio: 1.5% o.w.f) that had been dyed orange using an acidic dye (dye: Dystar Telon Orange AGT 01; C.I. Acid Orange 116) was cut and prepared as a colored polymer raw material having a square size of approximately 3 cm on one side.

### Raw Material 8

An undyed plain wool fiber fabric was cut and prepared as a colorless polymer raw material having a square size of approximately 3 cm on one side.

### Raw Material 9

A plain wool fiber fabric (dye weight ratio: 1.5% o.w.f) that had been dyed orange using an acidic dye (dye: Dystar Telon Orange AGT 01; C.I. Acid Orange 116) was cut and prepared as a colored polymer raw material having a square size of approximately 3 cm on one side.

### Raw Material 10

An undyed plain silk fiber fabric was cut and prepared as a colorless polymer raw material having a square size of approximately 3 cm on one side.

### Raw Material 11

A plain silk fiber fabric (dye weight ratio: 1.5% o.w.f) that had been dyed orange using an acidic dye (dye: Dystar Telon Orange AGT 01; C.I. Acid Orange 116) was cut and prepared as a colored polymer raw material having a square size of approximately 3 cm on one side.

### Raw Material 12

An undyed plain acrylic fiber fabric was cut and prepared as a colorless polymer raw material having a square size of approximately 3 cm on one side.

### Raw Material 13

A plain acrylic fiber fabric (dye weight ratio: 1.5% o.w.f) that had been dyed yellow using a basic dye (Dystar Astrazon Golden Yellow GL-E 200%; C.I. Basic Yellow 28) was cut and prepared as a colored polymer raw material having a square size of approximately 3 cm on one side.

### Raw Material 14

An undyed plain cotton fiber fabric was cut and prepared as a colorless polymer raw material having a square size of approximately 3 cm on one side.

### Raw Material 15

A plain cotton fiber fabric (dye weight ratio: 2.0% o.w.f) that had been dyed yellow using a reactive dye (Dystar Remazol Golden Yellow RNL; C.I. Reactive Orange 107) was cut and prepared as a colored polymer raw material having a square size of approximately 3 cm on one side.

### Raw Material 16

An undyed plain rayon fiber fabric was cut and prepared as a colorless polymer raw material having a square size of approximately 3 cm on one side.

### Raw Material 17

A plain rayon fiber fabric (dye weight ratio: 2.0% o.w.f) that had been dyed yellow using a reactive dye (Dystar Remazol Golden Yellow RNL; C.I. Reactive Orange 107) was cut and prepared as a colored polymer raw material having a square size of approximately 3 cm on one side.

### Reactivity Comparison Depending on Polymers and Extractant Type

### Example 1

Approximately 0.1 g of a navy (dye: Disperse Blue 56) polyester fiber of Raw Material 2 was placed in a 50 ml vial containing 10.0 g of anisole (methoxybenzene) previously heated and maintained at a temperature of 130°C and then stirred at 300 rpm for 10 minutes using a magnetic stirrer. Next, the polyester fiber was taken out using tweezers and washed by immersing the polyester fiber in 10.0 g of the same solvent which had been (anisole) prepared in a 50 ml vial and maintained at 90°C for about 1 to 2 seconds. Then, the polyester fiber was transferred to a glass evaporating dish. Afterward, the polyester fiber was placed in a vacuum dryer maintained at 60°C under vacuum conditions (≤ 2 mmHg) and dried for more than 12 hours to prepare a fiber from which a dye thereof was removed.

The color coordinate values (L*a*b*) for the fiber from which the dye thereof had been removed were measured using a spectrophotometer (manufacturer: Konica Minolta model name: CM-3600A) . The values expressed as L*a*b* were color space coordinate values standardized by the International Commission on Illumination (CIE). L* represented a value expressed from 0 (black) to 100 (white) indicating lightness. a* and b* represented values expressed on the complementary color axes for red (+)/green (-) and yellow (+)/blue (-), respectively. For the fiber from which the dye thereof had been removed and Raw Materials 1 to 17 specified above, the spectral spectra were measured using a near-infrared spectrophotometer (NIR).

### Example 2

A fiber was treated in the same manner as in Example 1, except that a red (dye: Disperse Red 1) fiber of Raw Material 3 was used as a polyester fiber raw material.

### Example 3

A fiber was treated in the same manner as in Example 1, except that a red (dye: Disperse Red 9) fiber of Raw Material 4 was used as a polyester fiber raw material.

### Example 4

A fiber was treated in the same manner as Example 1, except that a black (dye: Dystar Dianix Deep Black Plus) fiber blended with 82% polyester and 18% spandex from Raw Material 5 was used as a polymer fiber raw material containing an ester functional group.

### Example 5

A fiber was treated in the same manner as Example 1, except that an orange (dye: Acid Orange 116) nylon fiber of Raw Material 7 was used as a fiber raw material other than a polymer containing an ester functional group.

### Example 6

A fiber was treated in the same manner as Example 1, except that an orange (dye: Acid Orange 116) wool fiber as Raw Material 9 was used as a fiber raw material other than a polymer containing an ester functional group.

### Example 7

A fiber was treated in the same manner as Example 1, except that an orange (dye: Acid Orange 116) silk fiber as Raw Material 11 was used as a fiber raw material other than a polymer containing an ester functional group.

### Example 8

A fiber was treated in the same manner as Example 1, except that a yellow (dye: Basic Yellow 28) acrylic fiber as Raw Material 13 was used as a fiber raw material other than a polymer containing an ester functional group.

### Example 9

A fiber was treated in the same manner as Example 1, except that a yellow (dye: Reactive Orange 107) cotton fiber as Raw Material 15 was used as a fiber raw material other than a polymer containing an ester functional group.

### Example 10

A fiber was treated in the same manner as Example 1, except that a yellow (dye: Reactive Orange 107) rayon fiber as Raw Material 17 was used as a fiber raw material other than a polymer containing an ester functional group.

### Example 11

A fiber was treated in the same manner as Example 1, except that ethylbenzoate was used as an extractant.

### Example 12

A fiber was treated in the same manner as Example 1, except that ethoxybenzene was used as an extractant.

### Comparative Example 1

A fiber was treated in the same manner as Example 1, except that triethylphosphate was used as an extractant.

### Comparative Example 2

A fiber was treated in the same manner as Example 1, except that butylbutyrate was used as an extractant.

### Comparative Example 3

A fiber was treated in the same manner as Example 1, except that methoxycyclohexane was used as an extractant.

### Comparative Example 4

A fiber was treated in the same manner as Example 1, except that 1,4-xylene was used as an extractant.

### Comparative Example 5

A fiber was treated in the same manner as Example 1, except that morpholine was used as an extractant.

### Comparative Example 6

A fiber was treated in the same manner as Example 1, except that 1-methylpyrrolidin-2-one was used as an extractant.

### Comparative Example 7

A fiber was treated in the same manner as Example 1, except that methylsulfonylmethane was used as an extractant.

### Comparative Example 8

A fiber was treated in the same manner as Comparative Example 6 except that an orange (dye: Acid Orange 116) nylon fiber of Raw Material 7 was used as a fiber raw material other than a polymer containing an ester functional group.

### Comparative Example 9

A fiber was treated in the same manner as Comparative Example 6 except that an orange (dye: Acid Orange 116) wool fiber fabric of Raw Material 9 was used as a fiber raw material other than a polymer containing an ester functional group.

### Comparative Example 10

A fiber was treated in the same manner as Comparative Example 6 except that an orange (dye: Acid Orange 116) silk fiber fabric of Raw Material 11 was used as a fiber raw material other than a polymer containing an ester functional group.

### Comparative Example 11

A fiber was treated in the same manner as Comparative Example 6 except that a yellow (dye: Basic Yellow 28) acrylic fiber fabric of Raw Material 13 was used as a fiber raw material other than a polymer containing an ester functional group.

### Comparative Example 12

A fiber was treated in the same manner as Comparative Example 6 except that a yellow (dye: Reactive Orange 107) cotton fiber fabric of Raw Material 15 was used as a fiber raw material other than a polymer containing an ester functional group.

### Comparative Example 13

A fiber was treated in the same manner as Comparative Example 6 except that a yellow (dye: Reactive Orange 107) rayon fiber fabric of Raw Material 17 was used as a fiber raw material other than a polymer containing an ester functional group.

### Comparative Example 14

A fiber was treated in the same manner as Comparative Example 7 except that an orange (dye: Acid Orange 116) nylon fiber of Raw Material 7 was used as a fiber raw material other than a polymer containing an ester functional group.

### Comparative Example 15

A fiber was treated in the same manner as Comparative Example 7 except that an orange (dye: Acid Orange 116) wool fiber fabric of Raw Material 9 was used as a fiber raw material other than a polymer containing an ester functional group.

### Comparative Example 16

A fiber was treated in the same manner as Comparative Example 7 except that an orange (dye: Acid Orange 116) silk fiber fabric of Raw Material 11 was used as a fiber raw material other than a polymer containing an ester functional group.

### Comparative Example 17

A fiber was treated in the same manner as Comparative Example 7 except that a yellow (dye: Basic Yellow 28) acrylic fiber fabric of Raw Material 13 was used as a fiber raw material other than a polymer containing an ester functional group.

### Comparative Example 18

A fiber was treated in the same manner as Comparative Example 7 except that a yellow (dye: Reactive Orange 107) cotton fiber fabric of Raw Material 15 was used as a fiber raw material other than a polymer containing an ester functional group.

### Comparative Example 19

A fiber was treated in the same manner as Comparative Example 7 except that a yellow (dye: Reactive Orange 107) rayon fiber fabric of Raw Material 17 was used as a fiber raw material other than a polymer containing an ester functional group.

### Extractant Effect Comparison Depending on Fiber Type

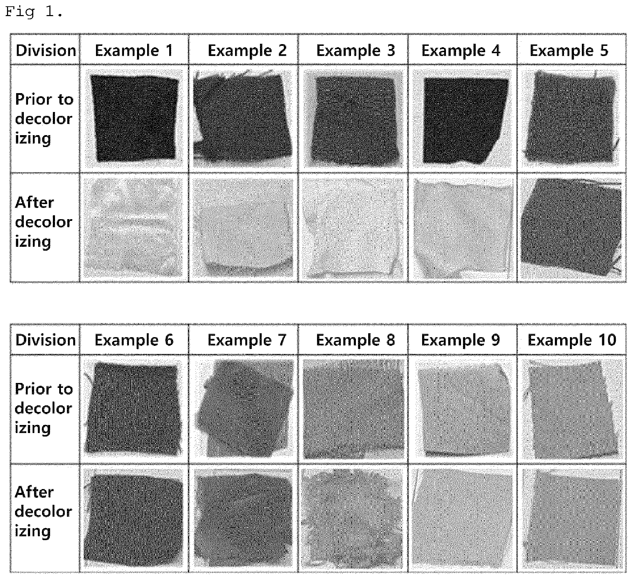

FIG. 1 shows the extent of dye removal from ester functional group-containing colored polymer fibers and colored fibers other than the ester functional group-containing colored polymer fibers by bringing the polymer fibers into contact with an extractant (anisole) according to Examples 1 to 10. In FIG. 1, photographs are shown of the fibers having undergone a decolorizing process, in which, dye was extracted by bringing various types of fabrics into contact with anisole which had been heated to 130°C for 10 minutes under the conditions specified in Example 1.

As shown in FIG. 1, after the decolorizing process with anisole, color-producing foreign substances were extracted from the ester functional group-containing polymer fibers. Meanwhile, no color-producing foreign substances contained in the fibers other than the ester functional group-containing polymer fibers were extracted.

After the decolorizing, the color of ester functional group-containing polymer fibers was clearly bright regardless of the color and lightness of the starting raw materials to the extent that the bright color thereof could be confirmed with the naked eye. It was confirmed that there was almost no color change in fibers other than ester functional group-containing polymer fibers.

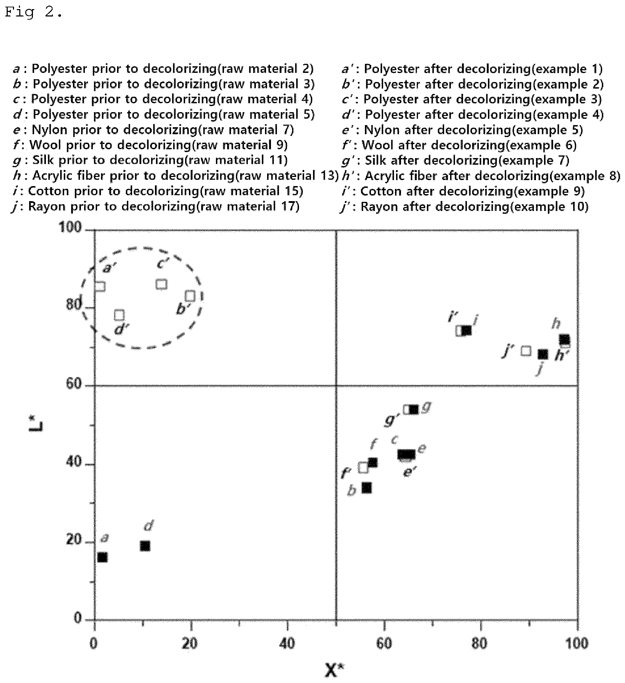

The color space coordinate values (L*a*b*) measured using a spectrophotometer for the fibers from which dye had been removed after the fibers had undergone a decolorizing process by being brought into contact with anisole in Examples 1 to 10 are shown in Table 1. FIG. 2 shows the two-dimensional coordinate values (L*X*) converted from color space coordinate values (L*a*b*) and plotted at plane coordinates.

**[Table 1]**

| Division | Raw material | Polymer type | Dye | Division | 'L* | a* | b* |
|---|---|---|---|---|---|---|---|
| Example 1 | Raw material 2 | Polyester | Disperse Blue 56 | Prior to decolorizing | 19.1 | 1.6 | -10.4 |
| | | | | After decolorizing | 85.5 | 0.7 | 0.7 |
| Example 2 | Raw material 3 | Polyester | Disperse Red 1 | Prior to decolorizing | 33.9 | 48.7 | 28.3 |
| | | | | After decolorizing | 83.0 | 16.4 | 11.0 |
| Example 3 | Raw material 4 | Polyester | Disperse Red 9 | Prior to decolorizing | 42.5 | 59.7 | 22.8 |
| | | | | After decolorizing | 86.0 | 13.9 | -0.1 |
| Example 4 | Raw material 5 | 82% polyester + Spandex 18% | Dystar Dianix Deep Black Plus | Prior to decolorizing | 16.1 | 0.2 | -1.6 |
| | | | | After decolorizing | 78.1 | 1.2 | 5.0 |
| Example 5 | Raw material 7 | Nylon | Acid Orange 116 | Prior to decolorizing | 42.3 | 44.3 | 48.1 |
| | | | | After decolorizing | 41.9 | 43.9 | 47.1 |
| Example 6 | Raw material 9 | Wool | Acid Orange 116 | Prior to decolorizing | 40.5 | 41.2 | 40.4 |
| | | | | After decolorizing | 39.2 | 40.4 | 38.1 |
| Example 7 | Raw material 11 | Silk | Acid Orange 116 | Prior to decolorizing | 54.0 | 40.6 | 52.2 |
| | | | | After decolorizing | 54.1 | 39.7 | 51.3 |
| Example 8 | Raw material 13 | Acrylic fiber | Basic Yellow 28 | Prior to decolorizing | 71.7 | 38.5 | 89.4 |
| | | | | After decolorizing | 71.2 | 40.0 | 88.9 |
| Example 9 | Raw material 15 | Cotton | Reactive Orange 107 | Prior to decolorizing | 74.1 | 26.9 | 72.3 |
| | | | | After decolorizing | 74.1 | 27.0 | 71.0 |
| Example 10 | Raw material 17 | Rayon | Reactive Orange 107 | Prior to decolorizing | 68.2 | 35.2 | 86.1 |
| | | | | After decolorizing | 68.9 | 33.4 | 82.8 |

As shown in Table 1 above, brightness values measured by spectrophotometry for the ester functional group-containing polymer fibers from which dyes had been removed according to Examples 1 to 4 were very high in a range of 78 to 86. The reason why the measured brightness values did not reach 100 is due to the fabric nature of the fibers and the unique characteristics of the measurement environment in which the dark cotton used as a measurement background came into play. Among color coordinate values, the a* and b* values expressed on the complementary color axes for red/green and yellow/blue were significantly different in fiber primary colors, but there was a clear tendency for the values to approach a neutral value below 17 after decolorizing.

Meanwhile, as shown in Table 1 above, brightness values for fibers (non-polyester) of Examples 5 to 10 after the decolorizing process, measured using a spectrophotometer were 74.1 or less. These values were almost the same as those of the initial raw materials that had not undergone the decolorizing process. In addition, the a* and b* values after dye removal were around 26 to 89, which were relatively far from the neutral value. Therefore, it was found that the decolorization effect caused by the extractant was limited to polyester functional group-containing polymer fibers.

In Example 4, the raw material was a polymer fiber blended with polyester and spandex. The fiber had a very dark color with a brightness value of about 16 before the decolorizing process, but the value was close to 78 after the decolorizing process. The a * and b * values for the fiber were 1.2 and 5.0, respectively, and a clear dye removal effect was observed. These results suggested that by using the extractant proposed in the present disclosure, color-producing foreign substances could be removed even from blended polymer fibers with ester functional groups (i.e., the blended polymer fibers include ester functional group-containing polymers and other types of fibers). Through this, it has been well demonstrated that the selecting yield of ester functional group-containing polymers could be improved. When color is developed in blended fibers containing highly concentrated ester functional group-containing polymers, disperse dyes are commonly used. This dye selectively dyes only ester functional group-containing polymers, so the dye may be provided in a method of selecting highly concentrated ester functional group-containing polymers through effective dye removal in a decolorizing process according to the present disclosure. At this point, the blended fibers including the highly concentrated ester functional groups may mean that ester functional group-containing polymer fibers may be included in an amount of 30 volume % or more, preferably 50 volume %, and more preferably 80 volume % or more.

As shown in FIG. 2, the two-dimensional coordinate values converted from the color space coordinate values (L*a*b*) for the ester functional group-containing polymer fibers, from which the dye had been removed after the decolorizing, are shown. Herein, L* was expressed on the vertical axis. X* was expressed on the horizontal axis, representing the square root of the sum of the squares of each of the a* and b* values. It was observed that L* satisfied a coordinate value criterion of 60 or more, and X* satisfied a coordinate value criterion of 50 or less at the same time.

From this, it was confirmed that relatively simple selection criteria could be presented by converting the color space coordinate values (L*a*b*) of fibers, from which the dye had been removed, into two-dimensional coordinate values (L*X*). Through this, it was confirmed that ester functional group-containing polymer fibers could be easily selected.

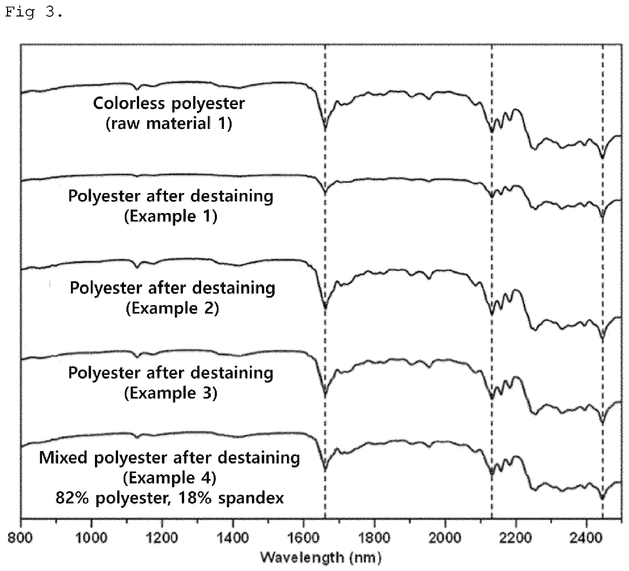

FIG. 3 shows differences in near-infrared spectral spectra depending on ester functional group-containing polymer fibers according to Examples 1 to 4 of the present disclosure. In FIG.3, the spectral spectrum results of selected fibers using a near-infrared spectrophotometer are shown. At this point, the selected fibers satisfied coordinate value criteria "L* of 60 or more and X* of 50 or less" at the same time, and the values meant two-dimensional coordinate values (L*X*) which were converted from the color space coordinate values (L*a*b*) measured using a spectrophotometer for the fibers from which the dye had been removed under the conditions of Example 1. When the spectral spectra of the fibers with coordinate values that satisfied the criteria were compared, peaks with the same characteristics (corresponding to the characteristic peaks of polyester) were observed.

### Decolorization Effect Comparison Depending on Extractant Type

In Examples 1, 11, 12, and Comparative Examples 1 to 5, the same conditions were applied except for the extractants used for decolorization to identify the decolorization effect by different extractants. The navy polyester fiber of Raw Material 2 was used as a fiber raw material. The temperature during the decolorizing process was kept constant at 130°C.

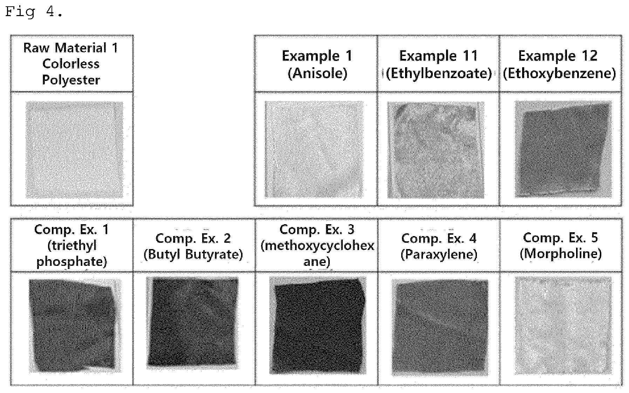

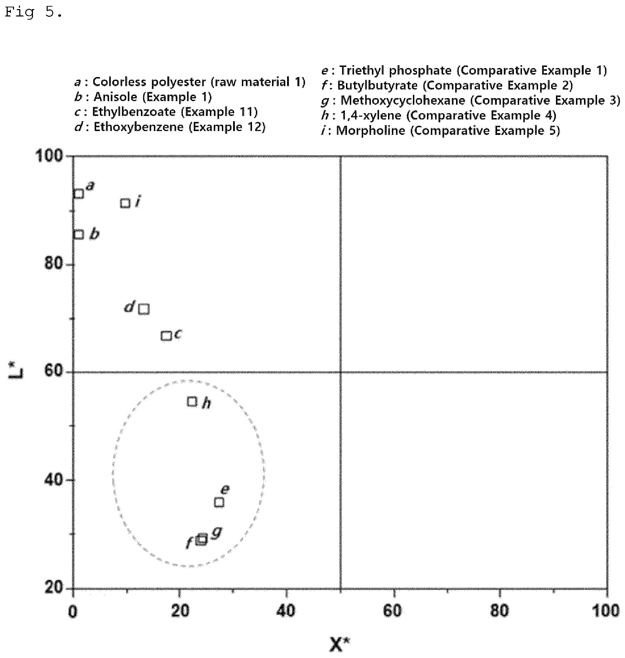

FIG. 4 shows photographs of the fibers after having undergone the decolorizing process in Examples 11 and 12 and Comparative Examples 1 to 5. Table 2 below shows the color space coordinate values (L*a*b*) measured using a spectrophotometer for each polyester fiber after the colorless polyester raw material of Raw Material 1 and Examples 11 and 12 and Comparative Examples 1 to 5 had undergone the decolorizing process. FIG. 5 shows two-dimensional coordinate values converted from the color space coordinate values (L*a*b*) shown in Table 2.

**[Table 2]**

| Division | Extractant type | L* | a* | b* |
|---|---|---|---|---|
| Raw material 1 | - | 93.1 | -0.5 | 0.9 |
| Example 1 | Anisole | 85.5 | 0.7 | 0.7 |
| Example 11 | Ethylbenzoate | 66.7 | -7.1 | -16.0 |
| Example 12 | Ethoxybenzene | 71.7 | -5.9 | -11.8 |
| Comparative Example 1 | Triethylphosphate | 36.0 | -4.3 | -27.1 |
| Comparative Example 2 | Butylbutyrate | 28.9 | -1.7 | -23.9 |
| Comparative Example 3 | Methoxycyclohexane | 29.3 | -1.9 | -24.1 |
| Comparative Example 4 | 1,4-xylene | 54.5 | -7.1 | -21.0 |
| Comparative Example 5 | Morpholine | 91.3 | -0.3 | 9.7 |

In Example 1, the brightness value (L*) was 85.5. The a* and b* values on the complementary color axis for the color were 0.7 and 0.7, respectively. These values were close to the color coordinate values of the colorless polyester fiber of Raw Material 1. In addition, ethyl benzoate and ethoxy benzene used in Examples 11 and 12 effectively removed color-producing foreign substances. Accordingly, it was confirmed that the color coordinate values for the fibers obtained after decolorizing with ethyl benzoate and ethoxy benzene were within the range of the criteria for the dye removal effect proposed in the present disclosure.

In Comparative Examples 1 to 4, partial decolorization of the fiber was observed, but the residual amount of dye was large enough to be easily confirmed with the naked eye. It was observed that the color coordinate values for the fibers after extractant treatment were 55 or less in brightness (L*), and the a* and b* values had large negative deviations (green and blue) from the neutral value.

In the case of removing dye by bringing ester functional group-containing colored polymer fibers into contact with the extractant according to the present disclosure, all brightness (L*) values measured by spectrophotometry were 60 or more, and the a* and b* values on the complementary color axis for the color were mostly measured close to the neutral value. Meanwhile, two-dimensional coordinates (L*X*) might more clearly present selection criteria to identify decolorizing performance when with values converted from measured color space coordinate values. When the conversion was carried out, decolorization effect was observed in a value range (L* of 60 or more, and X* of 50 or less) .

Methoxycyclohexane had a similar structure to anisole, which was one of the extractants according to the present disclosure, but the central functional group of the compound, methoxycyclohexane, was transformed into a saturated cycloalkane rather than an aromatic ring. When this was used as an extractant and applied under the same conditions as Example 1 (Comparative Example 3), as shown in FIG. 5, the dye removal effect was much lower than when anisole was applied as an extractant (Example 1).

In the case that the color space coordinate values (L*a*b*) measured using a spectrophotometer for Raw Material 1 and the fibers which were prepared by Examples 11, 12, and Comparative Examples 1 to 5 were converted into two-dimensional coordinate values (L*X*), when an extractant having one or more of the chemical structures proposed in the present disclosure was used (Examples 11 and 12), the two-dimensional coordinate values were within the criteria range (L* of 60 or more, and X* of 50 or less) . Meanwhile, in Comparative Examples 1 to 4 in which compounds with no aromatic ring structures as a central functional group were applied as an extractant, the color coordinate values for the fibers were outside the criteria range after decolorizing, showing that the compounds showed minimal effect as extractants.

As described above in the examples to compare the effects of extractants, when a substance having a chemical structure other than those of the extractant proposed in the present disclosure (Formula 1 or Formula 2) was applied as an extractant, a low decolorization effect was observed. This might be because the aromatic ring and the functional group bound thereto interacted with the ester functional group-containing polymers through a pi bond or hydrogen bond.

Anisole and methoxycyclohexane have the same number of carbon atoms and have a methoxy group bonded to the hydrocarbon ring. However, they are distinguished since anisole has an unsaturated hydrocarbon structure (aromatic compounds), and methoxycyclohexane has a saturated hydrocarbon structure (cyclohexane) in the form of a ring. Here in these experiments, Anisole (Example 1) and methoxycyclohexane (Comparative Example 3) were added at different concentrations to ester functional group-containing polymers, and then chemical shift changes in the mixture were observed using nuclear magnetic resonance (NMR) spectroscopy.

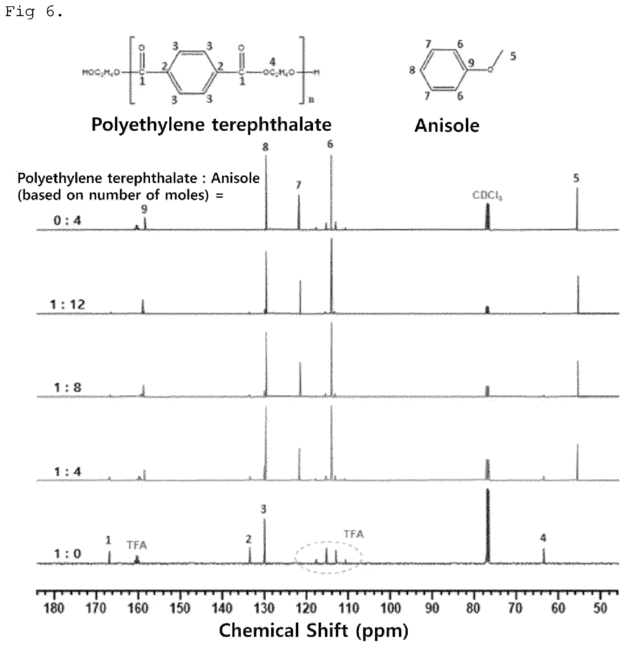

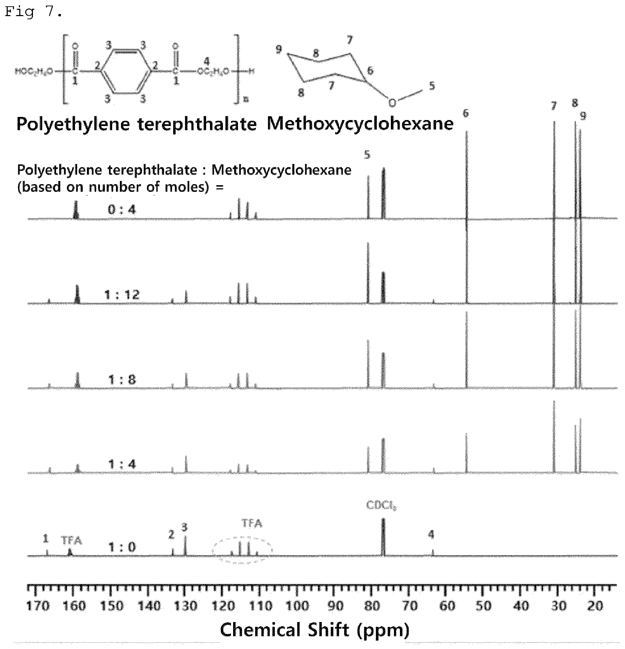

When the spectral results measured by ¹³C NMR (FIGs. 6 and 7) were compared, no chemical shifts were found in both cases. This suggested that there were no significant changes in the compound skeleton itself that made up the hydrocarbons.

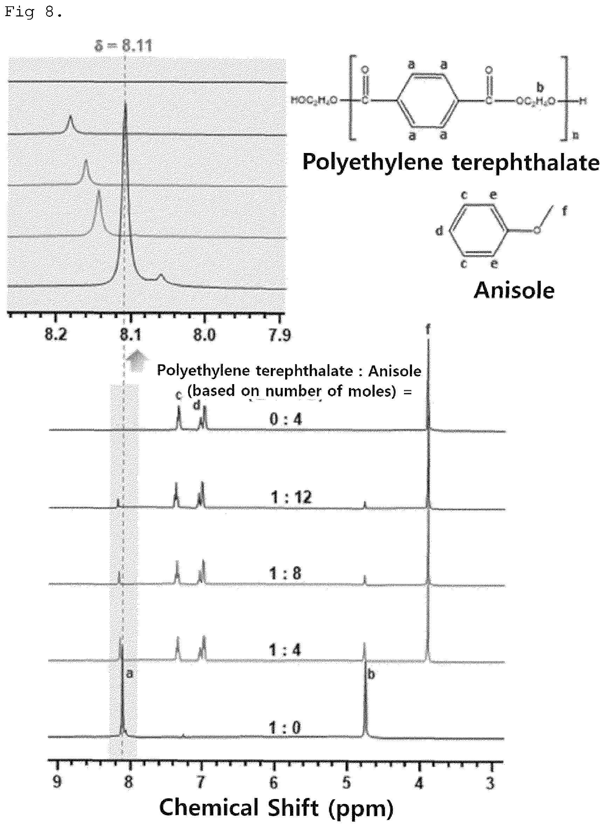

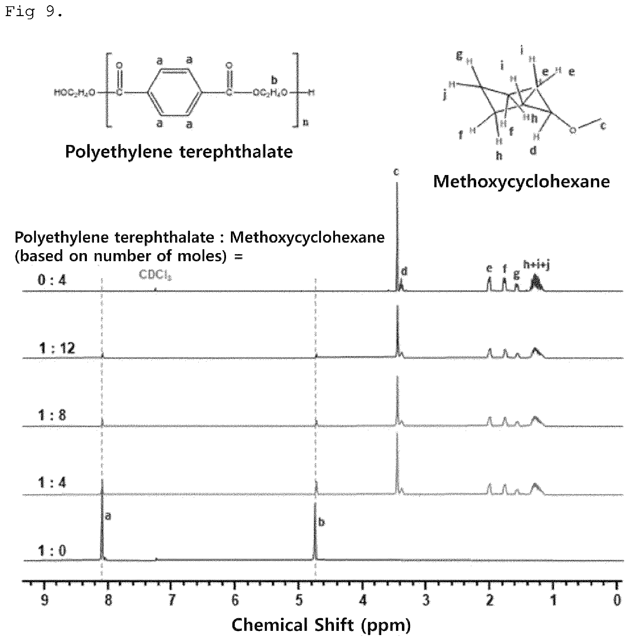

When the spectral results measured by ¹H NMR for the ester functional group-containing polymers in the sample containing each additive (FIGs. 8 and 9) were compared, no chemical shift was observed in the case of methoxycyclohexane used as an extractant (Comparative Example 3). Meanwhile, in the case of anisole used as an additive (Example 1), a high downfield chemical shift occurred along with a change in the electron density of hydrogen constituting the terephthalate functional group and the methylene functional group.

From this, it was inferred that the extractant having one or both of the chemical structures proposed in the present disclosure showed high interaction between ester functional group-containing polymers and heterogeneous molecules. It was also inferred that the addition of the highly concentrated extractant neutralized the weak physical bond maintained by the dye and polymers. Therefore, as the dye was irreversibly separated from the polymer matrix, the de-staining of the fibers progressed.

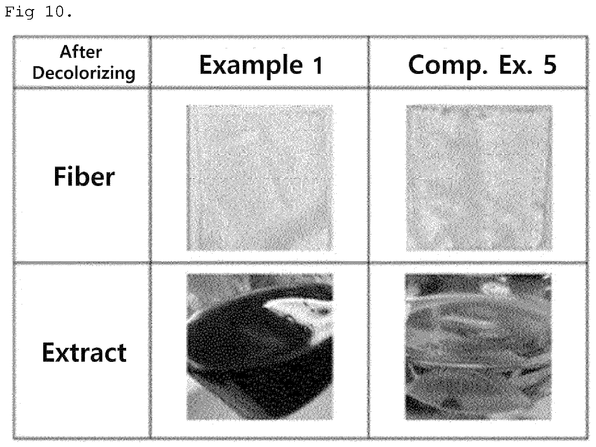

Morpholine of Comparative Example 5 showed a dye removal effect that satisfied the criteria "L* of 60 or more and X* of 50 or less" proposed in the present disclosure to select highly purified polyesters. However, morpholine reacted directly with the color-producing dye, causing decolorizing. Some of the components in the reactants were observed to remain in the polymer matrix. FIG. 10 shows the photographs of each of the solvents and polyester fibers after the de-staining process using anisole (methoxybenzene) used in Example 1 and morpholine used in Comparative Example 5.

In both Example 1 and Comparative Example 5, the navy polyester fibers of Raw Material 2 were used. However, in the case of using morpholine, the navy dye changed to light orange. After this color change, the fibers turned slightly orange, showing that the modified dye component remained. In Comparative Example 5, decolorizing occurred in the colored polyester fibers, but this was due to chemical modification of the dye chromophore. Thus, it was different from the expected effect of the present disclosure, in which the dye component was physically separated from the polymer matrix, and decolorizing occurred.

### De-staining Effect Comparison Depending on Extractant and Polymer Types

Comparative Examples 6 to 19 were performed to show a de-staining effect on ester functional group-containing polymer fibers and other fiber materials than the ester functional group-containing polymer fibers on condition that Comparative Examples 6 to 19 were subjected to the same extraction conditions as Experimental Example 1 using compounds but did not have the chemical structures proposed in the present disclosure. The types of polymers and extractants used in Comparative Examples 6 to 19, and the two-dimensional coordinate values (L*X*) showing the de-staining effect according to the extraction process are shown in Table 3 below.

**[Table 3]**

| Division | Raw material classification | Polymer type | Extractant type | L' | X* |
|---|---|---|---|---|---|
| Comparative Example 6 | Raw material 2 | Navy polyester | 1-methylpyrrolidin-2-one | 89.5 | 1.0 |
| Comparative Example 7 | Raw material 2 | Navy polyester | Methylsulfonylmethane | 88.0 | 1.0 |
| Comparative Example 8 | Raw material 7 | Orange nylon | 1-methylpyrrolidin-2-one | 58.2 | 62.8 |
| Comparative Example 9 | Raw material 9 | Orange wool | 1-methylpyrrolidin-2-one | 49.4 | 52.6 |
| Comparative Example 10 | Raw material 11 | Orange silk | 1-methylpyrrolidin-2-one | 88.9 | 14.6 |
| Comparative Example 11 | Raw material 13 | Yellow acrylic fiber | 1-methylpyrrolidin-2-one | - | - |
| Comparative Example 12 | Raw material 15 | Yellow cotton | 1-methylpyrrolidin-2-one | 74.1 | 75.8 |
| Comparative Example 13 | Raw material 17 | Yellow rayon | 1-methylpyrrolidin-2-one | 68.6 | 91.5 |
| Comparative Example 14 | Raw material 7 | Orange nylon | Methylsulfonylmethane | 50.7 | 66.6 |
| Comparative Example 15 | Raw material 9 | Orange wool | Methylsulfonylmethane | 44.0 | 50.5 |
| Comparative Example 16 | Raw material 11 | Orange silk | Methylsulfonylmethane | 85.6 | 20.7 |
| Comparative Example 17 | Raw material 13 | Yellow acrylic fiber | Methylsulfonylmethane | - | - |
| Comparative Example 18 | Raw material 15 | Yellow cotton | Methylsulfonylmethane | 74.2 | 76.6 |
| Comparative Example 19 | Raw material 17 | Yellow rayon | Methylsulfonylmethane | 66.4 | 83.8 |

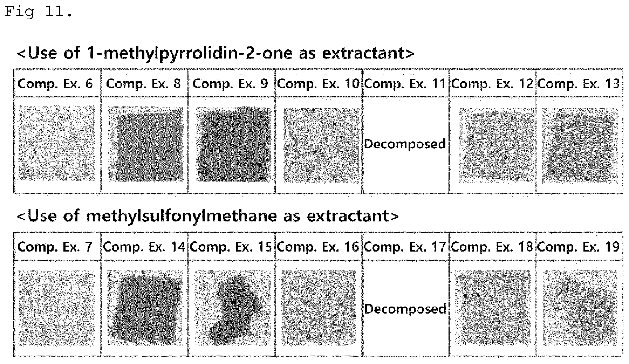

FIG. 11 shows the shape of the fibers after the same extraction process was applied to several fibers using extractants (1-methylpyrrolidin-2-one and methylsulfinylmethane) which did not have the chemical structures proposed in the present disclosure but showed a de-staining effect on ester functional group-containing polymers. When 1-methylpyrrolidin-2-one and methylsulfinylmethane were used as extractants for several types of polymers, decolorizing occurred in Comparative Examples 6 and 7 in which polyester was used. Among the cases in which fibers other than polyester fibers were used, fiber deformation was observed in all cases except for Comparative Examples 12, 13, and 18. In particular, the acrylic fiber was completely decomposed and could not be recovered.

The extractants used in Comparative Examples 6 to 19 which were not an extractant with one or more selected from the compounds represented by Formula 1 and the compounds represented by Formula 2 according to the present disclosure showed a de-staining effect on ester functional group-containing polymers. However, the extractants reacted or dissolved with other polymer types, making them difficult to apply in the sequential recycling process of polymers mixed with various materials. In addition, the extractants could also be difficult to use in the selecting process for selective separation and high purification of ester functional group-containing polymers.

These results explain well that some compounds that do not have the chemical structure of the compounds represented by Formula 1 or the compounds represented by Formula 2 proposed in the present disclosure are effective in decolorizing ester functional group-containing polymers but are not suitable for use as an extractant in the selecting process according to the present disclosure.

### De-staining Experiment When Mixed with Different Polymer Types

Considering that when the technique of the present disclosure was applied to an actual process, extractants were applied to a mixture of various types of polymers, the following experiments were performed after mixing all the same raw materials used in Examples 1 to 10.

### Example 13

0.3 g of each of ester functional group-containing polymer fibers of Raw Materials 2 to 5, and 0.3 g of each of the colored fibers other than the ester functional group-containing polymer fibers of Raw Materials 7, 9, 11, 13, 15, and 17 were mixed, and the fiber mixture was de-stained using a Soxhlet extraction method.

150g of anisole (methoxybenzene) was used as an extractant. After having been brought into contact with the extractant, the fibers were washed with ethanol, transferred to a glass evaporating dish, placed in a vacuum dryer maintained at 80°C under vacuum (≤ 2 mmHg), and dried for more than 12 hours.

With the fibers from which the dyes were removed, the color space coordinate values (L*a*b*) were measured using a spectrophotometer as in Example 1 and converted into two-dimensional coordinate values (L*X*). At this point, the fibers that satisfied criteria "L* of 60 or more and X* of 50 or less" were separated from the fibers that did not meet these criteria. Afterward, the spectral spectra of the separated fibers were measured using a near-infrared spectrophotometer.

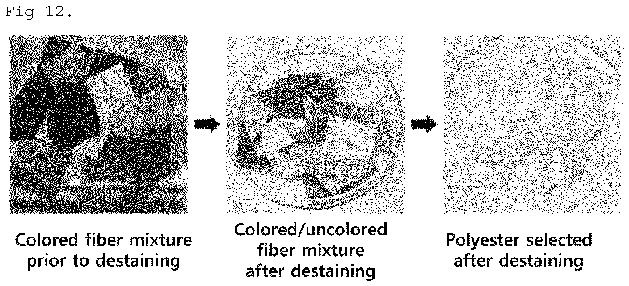

FIG. 12 shows the color changes of the colored fiber mixture before and after extractant treatment using a Soxhlet extractor. As shown in FIG. 12, when the fiber mixture and extractant were brought into contact with each other using the Soxhlet extractor, it was found that selective decolorizing occurred only in certain fibers (polyester fibers of Raw Materials 2 to 5).

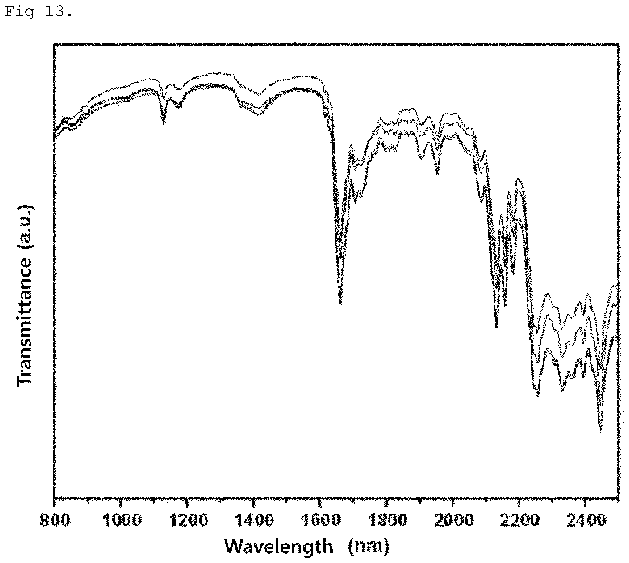

When the measurement results for the fibers using the spectrophotometer were obtained after the extraction process shown in FIG. 12, and then expressed in two-dimensional coordinate values (L*X*), fibers that satisfied the criteria "L* of 60 or more and X* of 50 or less" were selected. Then, the spectral spectra of the fibers were measured using a near-infrared spectrophotometer. The results are shown in FIG. 13.

As shown in FIG. 13, when the spectral spectra of the fibers with the criteria coordinate values (consistent with the spectra in FIG. 3) was examined, it was confirmed that all of the fibers were ester functional group-containing polymers.

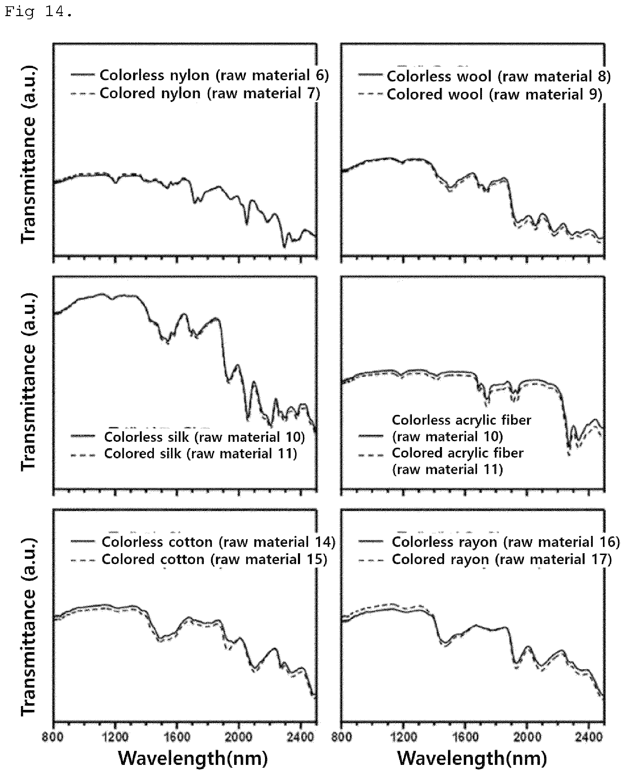

FIG. 14 shows spectra measured using a near-infrared spectrophotometer for fibers other than ester functional group-containing polymer fibers (nylon, wool, silk, acrylic, cotton, and rayon). When the fibers that had not been decolorized were randomly selected and the spectra thereof were measured using a near-infrared spectrophotometer, the measurements were observed to be one of the spectra in FIG. 14, not the spectra in FIG. 3 or FIG. 13. This clearly demonstrated that the previously described decolorizing process for colored fiber mixture might be significantly selective and efficient, and the process was performed to remove only disperse dyes dyed on ester functional group-containing fibers. As can be seen in FIG. 12, dyes previously introduced into each of the waste fibers might be used as indicators to chemically select ester functional group-containing polymers after the decolorizing process. Therefore, this technique has the advantage of not having to add a separate new material for selection after the decolorizing process.

As can be seen in FIGs. 12 to 14, when decolorizing using the extractant proposed in the present disclosure, selective decolorizing was possible only for ester functional group-containing polymer fibers even under fiber mixture conditions. It was confirmed that it was possible to classify and select ester functional group-containing polymer materials using only separable color discrimination criteria.

### Method of Preventing Exclusion of Colorless Polymers in Selection by Adding Staining Process to Raw Materials

Polymers discharged from waste include not only colored polymers but also white or transparent polymers. Thus, the following experiments were conducted to explore the possibility of selecting ester functional group-containing polymers from a mixture of colored and colorless polymers.

### Example 14

0.01 g of red disperse dye (Disperse Red 9) was added to a 30 ml vial containing 5.0 g of anisole and heated to 120°C. Approximately 0.1 g of the colorless polyester fiber of Raw Material 1 was added to a 30 ml vial containing anisole and red disperse dye (Disperse Red 9), kept for 2 minutes and then washed with ethanol. After dyeing, the washed fiber was transferred to a glass evaporation dish, placed in a vacuum dryer maintained at 60°C under vacuum (≤ 2 mmHg), and dried for more than 12 hours. The color space coordinate values (L*a*b*) for the fiber were measured using a spectrophotometer and then converted to two-dimensional coordinate values (L*X*), and the two-dimensional coordinate values are shown in Table 4.

### Comparative Example 20

Dyeing was carried out in the same manner as in Example 14 except that an undyed nylon fiber of Raw Material 6 was used as a fiber raw material other than an ester functional group-containing polymer fiber. The color space coordinate values (L*a*b*) for the fiber were measured using a spectrophotometer and then converted to two-dimensional coordinate values (L*X*), and the two-dimensional coordinate values are shown in Table 4.

### Comparative Example 21

Dyeing was carried out in the same manner as in Example 14 except that an undyed wool fiber of Raw Material 8 was used as a fiber raw material other than an ester functional group-containing polymer fiber. The color space coordinate values (L*a*b*) for the fiber were measured using a spectrophotometer and then converted to two-dimensional coordinate values (L*X*), and the two-dimensional coordinate values are shown in Table 4.

### Comparative Example 22

Dyeing was carried out in the same manner as in Example 14 except that an undyed silk fiber of Raw Material 10 was used as a fiber raw material other than an ester functional group-containing polymer fiber. The color space coordinate values (L*a*b*) for the fiber were measured using a spectrophotometer and then converted to two-dimensional coordinate values (L*X*), and the two-dimensional coordinate values are shown in Table 4.

### Comparative Example 23

Dyeing was carried out in the same manner as in Example 14 except that an undyed acrylic fiber of Raw Material 12 was used as a fiber raw material other than an ester functional group-containing polymer fiber. The color space coordinate values (L*a*b*) for the fiber were measured using a spectrophotometer and then converted to two-dimensional coordinate values (L*X*), and the two-dimensional coordinate values are shown in Table 4.

### Comparative Example 24

Dyeing was carried out in the same manner as in Example 14 except that an undyed cotton fiber of Raw Material 14 was used as a fiber raw material other than an ester functional group-containing polymer fiber. The color space coordinate values (L*a*b*) for the fiber were measured using a spectrophotometer and then converted to two-dimensional coordinate values (L*X*), and the two-dimensional coordinate values are shown in Table 4.

### Comparative Example 25

Dyeing was carried out in the same manner as in Example 14 except that an undyed rayon fiber of Raw Material 16 was used as a fiber raw material other than an ester functional group-containing polymer fiber. The color space coordinate values (L*a*b*) for the fiber were measured using a spectrophotometer and then converted to two-dimensional coordinate values (L*X*), and the two-dimensional coordinate values are shown in Table 4.

**[Table 4]**

| Division | Polymer type | Dyes used for re-dyeing | L* | X* |
|---|---|---|---|---|
| Example 14 | Colorless polyester | Disperse Red 9 | 73.0 | 36.7 |
| Comparative Example 2C | Colorless nylon | Disperse Red 9 | 86.5 | 12.1 |
| Comparative Example 21 | Colorless wool | Disperse Red 9 | 85.8 | 12.6 |
| Comparative Example 22 | Colorless silk | Disperse Red 9 | 92.3 | 15.1 |
| Comparative Example 23 | Colorless acrylic fiber | Disperse Red 9 | 93.8 | 2.7 |
| Comparative Example 24 | Colorless cotton | Disperse Red 9 | 91.1 | 6.2 |
| Comparative Example 25 | Colorless rayon | Disperse Red 9 | 92.0 | 3.6 |
| Example 15 | Colorless polyester | extracted waste dye | 48.8 | 5.5 |

The mixed solution of anisole and red disperse dye used in Example 14 and Comparative Examples 20 to 25 was simulated to be a waste extract containing disperse dye, which had been previously extracted from colored polyester fibers using an extractant. The extraction residual liquid obtained in the selectively decolorizing process described above in Examples 1 to 10 contained only disperse dyes selectively extracted from polyester fibers. Thus, the residual liquid differs only in color from the simulated solution. That is, the mixed solution and waste extract prepared by the simulation could show the same staining effect and have dyeing principle during the re-dyeing process.

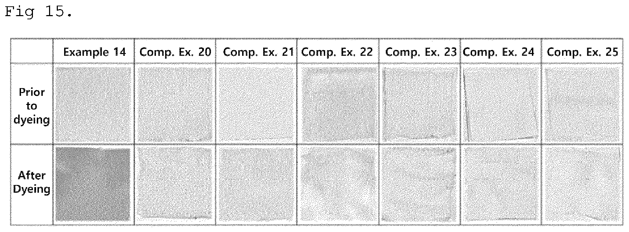

As shown in FIG. 15, when a visual inspection was immediately performed after the fibers were taken out from the vial, all fibers appeared to be stained. However, most of the dyes dyed on fibers other than polyester were removed after washing with ethanol.

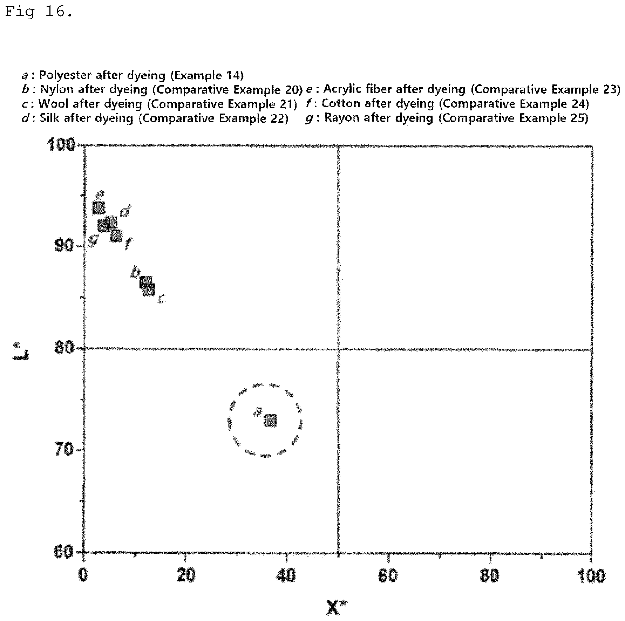

FIG. 16 shows two-dimensional coordinate values (L*X*) converted from the color space coordinate values and shown in Table 4 above. As shown in FIG. 16, when the dye-containing extractants and the fibers came into contact with each other by the methods of Example 14 and Comparative Examples 20 to 25, it was found that ester functional group-containing polymer fibers were selectively stained. It was confirmed that the dyeing effect was minimal for other fibers.

### Re-dyeing and Decolorizing Using Waste Extract Generated After Decolorizing

As described above, to selectively dye ester functional group-containing polymers in white or transparent polymer mixtures contained in waste, experiments were conducted in Example 15 and Comparative Examples 26 to 31, and the usability of the waste extractant generated after the decolorizing process included in the present disclosure was confirmed.

### Example 15

A dyeing process was performed under the same conditions as Example 14 and Comparative Examples 20 to 25 except for the use of anisole (hereinafter, referred to as waste extractant), which had been generated after the decolorizing process of the polymer mixture in Example 13 described above and had contained residual disperse dyes. The color space coordinate values (L*a*b*) for the experimental fiber were measured using a spectrophotometer and then converted into two-dimensional coordinate values (L*X*) . The value results are shown in Table 5. The undyed polyester fiber of Raw Material 1 was used as a fiber raw material. The fibers dried after dyeing were subjected to a decolorizing process under the same conditions as in Examples 1 to 10 above.

### Comparative Example 26

Dyeing and decolorizing were carried out in the same manner as in Example 15 except that an undyed nylon fiber of Raw Material 6 was used as a fiber raw material other than an ester functional group-containing polymer fiber. After the dyeing process, the color space coordinate values (L*a*b*) were measured to identify a fiber color change using a spectrophotometer and then converted to two-dimensional coordinate values (L*X*), and the values are shown in Table 5.

### Comparative Example 27

Dyeing and decolorizing were carried out in the same manner as in Example 15 except that an undyed wool fiber of Raw Material 8 was used as a fiber raw material other than an ester functional group-containing polymer fiber. After the dyeing process, the color space coordinate values (L*a*b*) were measured to identify a fiber color change using a spectrophotometer and then converted to two-dimensional coordinate values (L*X*), and the values are shown in Table 5.

### Comparative Example 28

Dyeing and decolorizing were carried out in the same manner as in Example 15 except that an undyed silk fiber of Raw Material 10 was used as a fiber raw material other than an ester functional group-containing polymer fiber. After the dyeing process, the color space coordinate values (L*a*b*) were measured to identify a fiber color change using a spectrophotometer and then converted to two-dimensional coordinate values (L*X*), and the values are shown in Table 5.

### Comparative Example 29

Dyeing and decolorizing were carried out in the same manner as in Example 15 except that an undyed acrylic fiber of Raw Material 12 was used as a fiber raw material other than an ester functional group-containing polymer fiber. After the dyeing process, the color space coordinate values (L*a*b*) were measured to identify a fiber color change using a spectrophotometer and then converted to two-dimensional coordinate values (L*X*), and the values are shown in Table 5.

### Comparative Example 30

Dyeing and decolorizing were carried out in the same manner as in Example 15 except that an undyed cotton fiber of Raw Material 14 was used as a fiber raw material other than an ester functional group-containing polymer fiber. After the dyeing process, the color space coordinate values (L*a*b*) were measured to identify a fiber color change using a spectrophotometer and then converted to two-dimensional coordinate values (L*X*), and the values are shown in Table 5.

### Comparative Example 31

Dyeing and decolorizing were carried out in the same manner as in Example 15 except that an undyed rayon fiber of Raw Material 16 was used as a fiber raw material other than an ester functional group-containing polymer fiber. After the dyeing process, the color space coordinate values (L*a*b*) were measured to identify a fiber color change using a spectrophotometer and then converted to two-dimensional coordinate values (L*X*), and the values are shown in Table 5.

**[Table 5]**

| Division | Polymer type | L' | X* |
|---|---|---|---|
| Example 15 | Colorless polyester | 48.8 | 5.5 |
| Comparative Example 26 | Colorless nylon | 81.9 | 16.3 |
| Comparative Example 27 | Colorless wool | 81.4 | 14.5 |
| Comparative Example 28 | Colorless silk | 91.3 | 6.5 |
| Comparative Example 29 | Colorless acrylic fiber | 94.2 | 3.6 |
| Comparative Example 30 | Colorless cotton | 84.1 | 14.8 |
| Comparative Example 31 | Colorless rayon | 91.5 | 4.9 |

The waste extractant used in the dyeing process in Example 15 and Comparative Examples 26 to 31 was the residual extractant generated during the selective decolorizing process of ester functional group-containing polymers in the polymer mixture previously described in detail in Example 13. The waste extractant contained only the disperse dye selectively extracted from polyester fibers. Therefore, the waste extractant differ from the simulated solution only in color. That is, the mixed solution and waste extractant prepared by the simulation could show the same staining effect and have dyeing principle during the re-dyeing process.

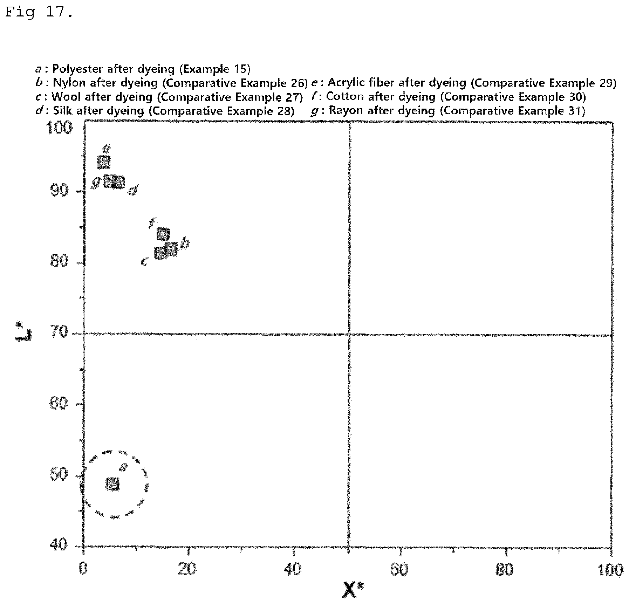

FIG. 17 shows two-dimensional coordinate values (L*X*) converted from the color space coordinate values and shown in Table 5 above. As shown in FIG. 17, when a dye-containing waste extractant, which had been generated during the decolorizing process, was brought into contact with fibers by the method of Example 15 and Comparative Examples 26 to 31, it was found that ester functional group-containing polymer fibers were selectively stained. It was confirmed that the dyeing effect was minimal for other fibers.

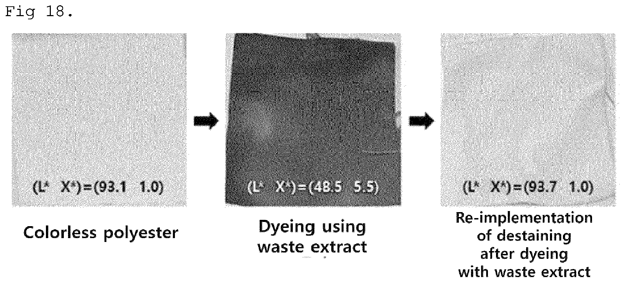

FIG. 18 shows the color change of polyester fibers re-dyed and decolorized through Example 15. As shown in FIG. 16, when re-dyeing or decolorizing fibers was carried out by the methods of Example 15 and Comparative Examples 26 to 31, it was possible to dye ester functional group-containing colorless polymer fibers by recycling the waste extractant. This shows that dyes introduced from the waste extractant might be effectively removed through the decolorizing technique included in the present disclosure.

From this, it was confirmed that when ester functional group-containing colorless polymers contained in the waste polymer mixture were selectively dyed, and then removing the dye was carried out again, it was possible to select ester functional group-containing polymers from a polymer mixture of various types of polymers, regardless of whether they were colored or colorless, by the method according to the present disclosure.

In addition, the residual extractant recovered in the chemical selecting process of the present disclosure contained highly concentrated disperse dyes capable of dyeing, so the disperse dyes might be reused in the dyeing process. Thus, an economical and eco-friendly waste fiber recycling process might be implemented.

As above, specific parts of the present disclosure have been described in detail. For those skilled in the art, this specific technique is described with merely preferred embodiments. It will be clear that the scope of the present disclosure is not limited by this. Accordingly, the substantial scope of the present disclosure will be defined by the appended claims and their equivalent.

## Claims

1. An extractant, selectively eluting and removing a color-producing foreign substance that selectively produces a color only in an ester functional group-containing polymer, from a polymer mixture comprising an ester functional group-containing colored polymer and another type of colored polymer,
wherein the extractant comprises one or more selected from compounds represented by Formula 1 and compounds represented by Formula 2 below, 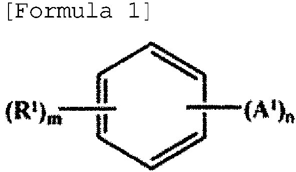 and 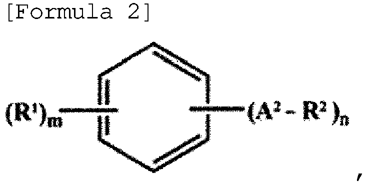
(wherein in Formula 1, A¹ comprises any one selected from the group consisting of a carboxyl group, an aldehyde group, and a nitrile group,
R¹ comprises any one selected from the group consisting of a hydroxyl group, carboxyl group, aldehyde group, nitrile group, straight-chain or branched saturated hydrocarbon having 1 to 6 carbon atoms, straight-chain or branched unsaturated hydrocarbon having 1 to 6 carbon atoms, cycloalkyl group having 4 to 6 carbon atoms, and aryl group having 6 to 12 carbon atoms,
m is any integer selected from a range of 0 to 5
wherein when the m is 2 or more, R¹ is the same or different from each other,
in addition, in Formula 2, A² comprises one or more selected from the group consisting of an ester group, an ether group, and a carbonyl group,
wherein R² is an alkyl group having 1 to 10 carbon atoms,
n is any integer selected from a range of 1 to 6,
wherein when the n is 2 or more, A² and R² are the same or different from each other,
other definitions of R¹ and m are the same as in Formula 1 above.)

2. The extractant of claim 1, wherein the compounds represented by Formula 1 or the compounds represented by Formula 2 comprises one or more selected from the group consisting of methoxybenzene, ethoxybenzene, butoxybenzene, 1,2-dimethoxybenzene, 1,3-dimethoxybenzene, 1,4-dimethoxybenzene, 1,2-diethoxybenzene, 1,3-diethoxybenzene, 1,4-diethoxybenzene, 1,2,3-trimethoxybenzene, 1,2,4-trimethoxybenzene, 1,3,5-trimethoxybenzene, 1,2,4-triethoxybenzene, 1,3,5-triethoxybenzene, 1,2,3,4-tetramethoxybenzene, 1,2,3,5-tetramethoxybenzene, 1,2,4,5-tetramethoxybenzene, 1-methoxy-2-methylbenzene, 1-methoxy-3-methylbenzene, 1-methoxy-4-methylbenzene, 1-ethyl-2-methoxybenzene, 1-ethyl-3-methoxybenzene, 1-ethyl-4-methoxybenzene, 1-ethoxy-2-methylbenzene, 1-ethoxy-3-methylbenzene, 1-ethoxy-4-methylbenzene, 1-ethoxy-2-ethylbenzene, 1-ethoxy-3-ethylbenzene, 1-ethoxy-4-ethylbenzene, 1-methoxy-2-prop-2-enylbenzene, 1-methoxy-3-prop-1-enylbenzene, 1-methoxy-3-prop-2-enylbenzene, 1-methoxy-4-prop-2-enylbenzene, 1-methoxy-4-[(E)-prop-1-enyl]benzene (cis), 1-methoxy-4-[(E)-prop-1-enyl]benzene (trans), methyl benzoate, ethyl benzoate, butyl benzoate, propyl benzoate, methyl2-ethoxybenzoate, methyl3-ethoxybenzoate, methyl4-ethoxybenzoate, ethyl2-ethoxybenzoate, ethyl3-ethoxybenzoate, ethyl4-ethoxybenzoate, ethyl(E)3-(4-hydroxy-3,5-dimethoxyphenyl)prop-2-enoate, 2,3-dimethoxy-4-methylbenzoic acid, 2,3-dimethoxybenzoic acid, 2,4-dimethoxybenzoic acid, 2,4-dihydroxybenzoic acid, 2,5-dimethoxy-4-methylbenzoic acid, 2,5-dimethoxybenzoic acid, 2,5-dihydroxybenzoic acid, 2,6-dimethoxy-4-methylbenzoic acid, 2,6-dimethoxybenzoic acid, 2,6-dihydroxybenzoic acid, 2-methoxybenzoic acid, 2-ethoxy-3-ethylbenzoic acid, 2-ethoxybenzoic acid, 2-hydroxy-3-methoxybenzoic acid, 2-hydroxy-4,5-dimethoxybenzoic acid, 2-hydroxy-4,6-dimethoxybenzoic acid, 2-hydroxy-4-methoxybenzoic acid, 2-hydroxy-5-methoxybenzoic acid, 2-hydroxy-6-methoxybenzoic acid, 2-hydroxybenzoic acid, 3-(4-hydroxy-3,5-dimethoxyphenyl)prop-2-enoic acid, 3,4-dimethoxybenzoic acid, 3,4-dihydroxybenzoic acid, 3,5-dimethoxy-4-methylbenzoic acid, 3,5-dimethoxybenzoic acid, 3-methoxybenzoic acid, 3-ethoxy-4-hydroxybenzoic acid, 3-ethoxy-5-ethylbenzoic acid, 3-ethoxybenzoic acid, 3-hydroxy-4,5-dimethoxybenzoic acid, 3-hydroxy-4-methoxybenzoic acid, 3-hydroxy-5-methoxybenzoic acid, 3-hydroxybenzoic acid, 4-methoxybenzoic acid, 4-ethoxy-2-ethylbenzoic acid, 4-ethoxy-3-ethylbenzoic acid, 4-ethoxybenzoic acid, 4-hydroxy-2,6-dimethoxybenzoic acid, 4-hydroxy-2,6-dimethylbenzoic acid, 4-hydroxy-2-methoxybenzoic acid, 4-hydroxy-3,5-dimethoxybenzoic acid, 4-hydroxy-3,5-dimethylbenzoic acid, 4-hydroxy-3-methoxybenzoic acid, 4-hydroxybenzoic acid, ethyl 2-methoxybenzoic acid, ethyl 3-methoxybenzoic acid, ethyl 4-methoxybenzoic acid, 2,3-dimethoxyphenol, 2,4-dimethoxyphenol, 2,5-dimethoxyphenol, 2,6-dimethoxy-4-prop-2-enylphenol, 2,6-dimethoxyphenol, 2-[(E)-prop-1-enyl]phenol, 2-methoxy-4-(prop-1-enyl)phenol, 2-methoxy-4-prop-2-enylphenol, 2- methoxyphenol, 2-ethoxyphenol, 2-tert-butyl-4-methoxyphenol, 3,4-dimethoxyphenol, 3,5-dimethoxyphenol, 3-methoxyphenol, 3-ethoxyphenol, 3-tert-butyl-4-methoxyphenol, 3-prop-1-en-2-ylphenol, 4-(prop-1-en-2-yl)phenol, 4 -(prop-2-en-1-yl)phenol, 4-methoxy-2-[(E)-prop-1-enyl]phenol, 4-methoxyphenol, 4-ethoxyphenol, 4-ethyl-2,3-dimethylphenol, 4-ethyl-2,5-dimethoxyphenol, 4-ethyl-2,6-dimethoxyphenol, 4-tert-butyl-2-methoxyphenol, 1-(2-hydroxy-4,6-dimethylphenyl)ethanone, 1-(4-methoxyphenyl)ethanone 1-(4-hydroxy-2,6-dimethoxyphenyl)ethanone, 1-(4-hydroxy-3,5-dimethoxyphenyl)ethanone, 1-(5-hydroxy-2,4-dimethylphenyl)ethanone, 1-phenylbutan-1-one 1-phenylethanone, 1-phenylpropan-1-one 2,3-dihydroxybenzaldehyde, 2,4-dihydroxybenzaldehyde, 2,5-dihydroxybenzaldehyde, 2-methoxybenzaldehyde, 2-methylbenzaldehyde, 2-butoxybenzaldehyde, 2-ethoxybenzaldehyde, 2-hydroxy-3,5-dimethoxybenzaldehyde, 2-hydroxy-3-methoxybenzaldehyde, 2-hydroxy-4,6-dimethoxybenzaldehyde, 2-hydroxy-4-methoxybenzaldehyde, 2-hydroxy-5-methoxybenzaldehyde, 2-hydroxy-6-methoxybenzaldehyde, 2-hydroxybenzaldehyde, 3,4-dihydroxybenzaldehyde, 3,5-dihydroxybenzaldehyde, 3-methoxybenzaldehyde, 3-methylbenzaldehyde, 3-butoxybenzaldehyde, 3-ethoxy-2-hydroxybenzaldehyde, 3-ethoxybenzaldehyde, 3-hydroxy-2-methoxybenzaldehyde, 3-hydroxy-4-methoxybenzaldehyde, 3-hydroxy-5-methoxybenzaldehyde, 3-hydroxybenzaldehyde, 4-methoxybenzaldehyde, 4-methylbenzaldehyde, 4-butoxybenzaldehyde, 4-ethoxybenzaldehyde, 4-hydroxy-2,6-dimethoxybenzaldehyde, 4-hydroxy-2-methoxybenzaldehyde, 4-hydroxy-3,5-dimethoxybenzaldehyde, 4-hydroxy-3-methoxybenzaldehyde, 4-hydroxybenzaldehyde, benzaldehyde, 2,3-dihydroxybenzonitrile, 2,4-dihydroxybenzonitrile, 2,5-dihydroxybenzonitrile, 2,6-dihydroxybenzonitrile, 2-methoxybenzonitrile, 2-methylbenzonitrile, 2-hydroxybenzonitrile, 3,4-dihydroxybenzonitrile, 3-methoxybenzonitrile, 3-methylbenzonitrile, 3-hydroxybenzonitrile, 4-methoxybenzonitrile, 4-methylbenzonitrile, 4-acetylbenzonitrile, 4-hydroxybenzonitrile, and benzonitrile.

3. A method of selecting an ester functional group-containing polymer from a colored polymer mixture of an ester functional group-containing colored polymer and other types of colored polymers, the method comprising:
(a) selectively decolorizing an ester functional group-containing colored polymer by bringing a polymer mixture of the ester functional group-containing colored polymer and other types of colored polymers into contact with an extractant that extracts a color-producing foreign substance; and
(b) separating the decolorized polymer from non-decolorized polymers in the step (a) by color difference.

4. The method of claim 3, wherein the ester functional group-containing polymer has a fiber or fabric form, and
wherein the fabric is an ester functional group-containing polyester fiber alone, or a blended fiber comprising an ester functional group-containing polyester fiber and one or more fibers selected from the group consisting of polyethylene, polypropylene, polystyrene, and polyvinyl chloride, cotton, linen, wool, rayon, acetate, acrylic, nylon, and spandex.

5. The method of claim 3, wherein the colored polymers are polymers colored by a foreign substance that produces one or more colors, and
wherein when the color space coordinate values (L*a*b*) for the polymer mixture measured using a spectrophotometer are converted to two-dimensional coordinate values (L*X* (X* represents the square root of the sum of the squares of each of a* and b* values)), a polymer that satisfies coordinate value criteria "L* of 60 or more and X* of 50 or less" is excluded.

6. The method of claim 3, wherein the decolorizing process in the step (a) is carried out in a temperature range of 70°C to 200°C.

7. The method of claim 3, wherein the decolorized polymer satisfies coordinate value criteria "L* of 60 or more and X* of 50 or less" when the color space coordinate values (L*a*b*) for the decolorized polymer in the step (b) are converted to two-dimensional coordinate values (L*X* (X* represents the square root of the sum of the squares of each of the a* and b* values) .

8. A method of selecting an ester functional group-containing polymer from a polymer mixture of the ester functional group-containing polymer and other types of polymers, the method comprising:
(i) selectively dyeing only an ester functional group-containing polymer by bringing a polymer mixture of the ester functional group-containing polymer and other types of polymers into contact with a color-producing foreign substance;
(ii) excluding colorless polymers from the polymer mixture by color difference after the step (i);
(iii) selectively decolorizing only an ester functional group-containing colored polymer by bringing the mixture obtained after the step (ii) into contact with an extractant that extracts a color-producing foreign substance; and
(iv) separating the decolorized polymers and non-decolorized polymers from each other by color difference.

9. The method of claim 8, wherein, in the step (ii), the polymers that satisfy coordinate value criteria "L* of 60 or more and X* of 50 or less" are excluded when color space coordinate values (L*a*b*) of the polymers are converted to two-dimensional coordinate values (L*X* (X* represents the square root of the sum of the squares of each of a* and b* values).

10. The method of claim 8, wherein, in the step (iii), the decolorized polymers that satisfy coordinate value criteria "L* of 60 or more and X* of 50 or less" are selected when the color space coordinate values (L*a*b*) of the decolorized polymers are converted to two-dimensional coordinate values (L*X* (X* represents the square root of the sum of the squares of each of a* and b* values) .

11. The method of claim 8, wherein a waste extractant, which is a mixture of the extractant and the color-producing foreign substance eluted during the decolorizing in the step (iii) is recovered and used during the dyeing in the step (i).
